# EUROPEAN PATENT APPLICATION

(11) **EP 3 797 834 A1**
(43) Date of publication of application: **31.03.2021**
(21) Application number: 20198246.9
(22) Date of filing: 24.09.2020
(51) Int. Cl.: A61P 35/04, A61K 31/337, A61K 31/573

(54) **CABAZITAXEL IN MCRPC PATIENTS PREVIOUSLY TREATED WITH DOCETAXEL AND WHO FAILED A PRIOR ANDROGEN SIGNALING TARGETED INHIBITOR AGENT**

(30) Priority: 25.09.2019 US 201962905652 P; 27.01.2020 US 202062966263 P; 12.05.2020 US 202063023471 P
(71) Applicant: Sanofi Mature IP, 75008 Paris (FR)
(72) Inventor: BENSFIA, Samira, Bridgewater, NJ 08807 (US)
(74) Representative: J A Kemp LLP

(57) **Abstract**

Provided herein are methods of using cabazitaxel in mCRPC patients previously treated with docetaxel and who failed a prior androgen signaling targeted inhibitor agent.

## Description

The present application relates to cabazitaxel in metastatic castration-resistant prostate cancer (mCRPC) patients previously treated with docetaxel and who failed a prior androgen signaling targeted inhibitor within 12 months.

Prostate cancer is the second leading cause of cancer-related death among men in the United States and the third leading cause of cancer deaths in Europe.

Treatment of advanced prostate cancer is palliative. Androgen ablation remains the mainstay of treatment, producing a rapid decrease in bone pain, metastases, and prostate-specific antigen (PSA) levels. Nevertheless, in virtually all patients, the tumor becomes resistant to castration within a median of 18 months after castration.

Four different classes of medical treatments have improved survival from metastatic castration-resistant prostate cancer (mCRPC) including taxanes (docetaxel/prednisone, cabazitaxel/prednisone), androgen signaling targeted inhibitors (abiraterone acetate, enzalutamide), immunotherapy (sipuleucel-T) and a bone-targeted radiopharmaceutical (radium-223 chloride). The therapeutic landscape has shifted towards treatment with life-extending therapies during earlier stages of disease. Docetaxel, abiraterone, enzalutamide and apalutamide, in combination with androgen deprivation therapy, improved survival in metastatic hormone-sensitive prostate cancer. Androgen signaling targeted inhibitors have also improved metastasis-free survival vs. placebo in patients with non-metastatic castration-resistant prostate cancer.

The challenge for physicians is now to integrate this broad armamentarium rationally in daily practice and appropriately tailor therapy to optimize treatment outcomes.

Metastatic CRPC is a heterogeneous disease with coexistence of cells sensitive and resistant to androgen signaling targeted inhibitors in a same patient. So, not all patients benefit from available treatments to the same extent. In the COU AA 301 study, about 30% of patients experienced primary resistance to abiraterone acetate/prednisone as evidenced by imaging-based progression at the first visit planned in the protocol - 3 months(De Bono al., N. Engl. J Med 2011; 364(21): 1995-2005; Fizazi et al., Lancet Oncol 2012;13(10): 983-92). In the AFFIRM study, about 25% of patients experienced imaging-based progression with enzalutamide at 3 months (Scher Het al., N Engl J Med 2012; (13)367:1187-97).

Docetaxel is the first-line chemotherapy for mCRPC according to international guidelines (e.g., National Comprehensive Cancer Networks® (NCCN) guidelines, or European Association of Urology guidelines (EAU)). However, many physicians currently prescribe an androgen signaling targeted inhibitor in sequence either before or after docetaxel. It is not known if prescribing docetaxel between treatments with two different androgen signaling targeted inhibitors is able to restore some sensitivity to androgen signaling targeted inhibitors.

The efficacy of cabazitaxel in mCRPC has been demonstrated in the TROPIC trial, an international, open label Phase III trial in which 755 patients with mCRPC who progressed during or after docetaxel were randomized to receive either intravenous cabazitaxel 25 mg/m² over 1 hour (N = 378) or mitoxantrone 12 mg/m² over 1530 minutes (N = 377) every 3 weeks for ten cycles, in combination with prednisone 10 mg daily. The trial met its primary endpoint, with a statistically significant (p <0.0001) improvement in median overall survival in patients receiving cabazitaxel (15.1 months; 95% CI, 14.1-16.3 months) compared to patients receiving mitoxantrone (12.7 months; 95% CI, 11.6-13.7 months). Cabazitaxel is approved for the treatment of mCRPC previously treated with a docetaxel-containing regimen.

Androgen signaling targeted inhibitors and docetaxel are commonly used in earlier stages of the disease, and it is likely that most patients diagnosed with metastatic prostate cancer will have received both, in either order.

Although patients with mCRPC have several treatment options, few reliable and significant data inform about the optimized treatment sequence. In practice, many patients receive androgen signaling targeted inhibitors in sequence, due to lack of level 1 evidence for cross-resistance between these agents and because they are available orally.

A number of studies have evaluated cohorts of patients receiving specific sequences of different AR-targeted agents in everyday clinical practice, but these studies do not provide any robust evidence capable of indicating the superiority of one AR-targeted-based sequence over another. The lack of any prospective trials assessing the activity of sequentially administered AR-targeted agents and the impossibility of translating the results of the pivotal trials into the current clinical context means that real-world experience is still the only source of information about the activity of AR-targeted agents administered after a second line of treatment. Cabazitaxel, abiraterone, and enzalutamide have all been tested in patients progressing after docetaxel treatment and have been found to be effective in prolonging overall survival; these drugs have not been directly compared in head-to-head trials, and no definite conclusions can be drawn concerning the superiority of one over the other (Caffo et al., Future Oncology, 15(25), pp. 2967-2962, 2019).

There is thus an urgent need to identify the best treatment sequences for mCRPC patients. It was here investigated whether cabazitaxel is superior to an androgen signaling targeted inhibitor in patients previously treated with docetaxel and an alternative androgen signaling targeted inhibitor. Surprisingly, it has now been demonstrated herein that cabazitaxel significantly improves important patient outcomes as compared to abiraterone acetate or enzalutamide. Specifically, the study described herein demonstrates that cabazitaxel more than doubled radiographic progression-free survival, and this benefit was observed across all pre-specified subgroups, irrespective of the previous alternative AR pathway inhibitor timing, before or after docetaxel. All other secondary end points (progression-free survival, PSA response, tumor response, pain response and time to symptomatic skeletal events) also favored cabazitaxel.

### DESCRIPTION OF THE INVENTION

Accordingly, provided herein is cabazitaxel, or a hydrate or solvate thereof, for use in a method of increasing progression free survival in a patient in need thereof, comprising administering an effective amount of cabazitaxel, or a hydrate or solvate thereof, to said patient having metastatic castration-resistant prostate cancer previously treated with docetaxel and who failed a prior AR-targeted agent.

In another embodiment provided herein is cabazitaxel, or a hydrate or solvate thereof, for use in a method of increasing overall survival in a patient in need thereof, comprising administering an effective amount of cabazitaxel, or a hydrate or solvate thereof, to said patient having metastatic castration-resistant prostate cancer previously treated with docetaxel and who failed a prior AR-targeted agent.

In another embodiment, provided herein is cabazitaxel, or a hydrate or solvate thereof, for use in reducing or removing pain in a patient having metastatic castration-resistant prostate cancer previously treated with docetaxel and who failed a prior AR-targeted agent. Reducing or removing pain may also be considered treatment of pain. Reducing or removing pain may involve a reduction in pain response and/or it may involve increasing the time to pain progression.

Also described herein is a method of increasing progression free survival in a patient in need thereof, comprising administering a clinically proven effective amount of cabazitaxel, or a hydrate or solvate thereof, to said patient having metastatic castration-resistant prostate cancer previously treated with docetaxel and who failed a prior AR-targeted agent.

Also described herein is a method of treating a patient having metastatic castration-resistant prostate cancer previously treated with docetaxel who failed a prior AR-targeted agent, comprising administering to said patient cabazitaxel, or a hydrate or solvate thereof, in an amount effective to increase progression free survival.

Also described herein is a method of treating a patient having metastatic castration-resistant prostate cancer previously treated with docetaxel who failed a prior AR-targeted agent, comprising administering to said patient cabazitaxel, or a hydrate or solvate thereof, in an amount effective to increase overall survival.

Also provided herein is cabazitaxel, or a hydrate or solvate thereof, for use in combination with a corticoid for use as a medicament in the treatment of patients having metastatic castration-resistant prostate cancer previously treated with docetaxel and who failed a prior AR-targeted agent.

Also provided herein is cabazitaxel, or a hydrate or solvate thereof, for use in combination with a corticoid for use as a medicament in the treatment of patients having metastatic castration-resistant prostate cancer previously treated with docetaxel and who failed a prior AR-targeted agent, wherein said combination increases progression free survival.

Also provided herein is cabazitaxel, or a hydrate or solvate thereof, for use in combination with a corticoid for use as a medicament in the treatment of patients having metastatic castration-resistant prostate cancer previously treated with docetaxel and who failed a prior AR-targeted agent, wherein said combination increases overall survival.

In some aspects, cabazitaxel, or a hydrate or solvate thereof, is administered in combination with a corticoid, for example prednisone or prednisolone. In some embodiments, this corticoid is administered at a daily dose of 10 mg orally.

In some aspects, cabazitaxel or a hydrate or solvate thereof is administered at a dose (defined for each administration) of 15 to 25 mg/m², for example at a dose of 15 mg/m², 20 mg/m², or 25 mg/m².

Cabazitaxel may be in the form of an acetone solvate. More particularly, the acetone solvate of cabazitaxel contains between 5% and 8% and, in some aspects, between 5% and 7% by weight of acetone.

In some aspects, cabazitaxel or a hydrate or solvate thereof may be administered by intravenous infusion at a dose of 15 to 25 mg/m², this administration cycle of the antitumor agent being repeated at an interval of 3 weeks between each cabazitaxel administration, which interval may be prolonged by 1 to 2 weeks depending on the tolerance to the preceding cabazitaxel administration.

In some aspects, cabazitaxel, or hydrate or solvate thereof, is administered prior to the administration of an alternative AR-targeted agent. Where an alternative AR-targeted agent is administered after cabazitaxel, the alternative AR-targeted agent is typically administered following disease progression after cabazitaxel therapy. For example, the alternative AR-targeted therapy may be administered within 12 months of said disease progression, or within 6 months of said disease progression.

In some aspects, the patient has not previously received an alternative AR-targeted agent.

In some aspects, the prior AR-targeted agent is abiraterone acetate and the alternative AR-targeted agent is enzalutamide. The patient may have been treated with docetaxel before treatment with abiraterone acetate. Alternatively, the patient may have been treated with docetaxel after failure of abiraterone acetate.

Thus, in some aspects, cabazitaxel or a hydrate or solvate thereof is provided for use (optionally in combination with a corticoid) in the treatment of patients having metastatic castration-resistant prostate cancer previously treated with docetaxel and who, subsequent to docetaxel (but before cabazitaxel therapy), failed prior abiraterone acetate therapy. Such patients may be administered enzalutamide subsequent to administration of cabazitaxel, e.g. after disease progression following cabazitaxel.

In alternative aspects, cabazitaxel or a hydrate or solvate thereof is provided for use (optionally in combination with a corticoid) in the treatment of patients having metastatic castration-resistant prostate cancer previously treated with docetaxel and who, prior to administration of docetaxel, failed abiraterone acetate therapy. Such patients may be administered enzalutamide subsequent to administration of cabazitaxel, e.g. after disease progression following cabazitaxel.

In other aspects, the prior AR-targeted agent is enzalutamide and the alternative AR-targeted agent is abiraterone acetate. The patient may have been treated with docetaxel before treatment with enzalutamide. Alternatively, the patient may have been treated with docetaxel after failure of enzalutamide.

Thus in some aspects, cabazitaxel or a hydrate or solvate thereof is provided for use (optionally in combination with a corticoid) in the treatment of patients having metastatic castration-resistant prostate cancer previously treated with docetaxel and who, subsequent to docetaxel (but before cabazitaxel therapy), failed prior enzalutamide therapy. Such patients may be administered abiraterone acetate subsequent to administration of cabazitaxel, e.g. after disease progression following cabazitaxel.

In alternative aspects, cabazitaxel or a hydrate or solvate thereof is provided for use (optionally in combination with a corticoid) in the treatment of patients having metastatic castration-resistant prostate cancer previously treated with docetaxel and who, prior to administration of docetaxel, failed enzalutamide therapy. Such patients may be administered abiraterone acetate subsequent to administration of cabazitaxel, e.g. after disease progression following cabazitaxel.

In particular aspects, the failure of a prior AR-targeted agent occurred within 12 months. In some aspects, the failure of a prior AR-targeted agent occurred within 6 months.

In some aspects, the patient having metastatic castration-resistant prostate cancer is aged 70 years or over, in particular the patient may be aged over 75 years. Such patients are a more challenging patient group in which to achieve successful outcomes such as increased overall survival, increased progression free survival and improved pain response.

In some aspects, the patient having metastatic castration-resistant prostate cancer has a high neutrophil count. Typically the neutrophil count is greater than 1.5 x 10⁹/L, for example 4.5 x 10⁹/L or greater; or 5.0 x 10⁹/L or greater. For example, the neutrophil count may be from 1.5 to 11 x 10⁹/L, for example from 5.0 to 11 x 10⁹/L.

In some aspects, the patient has a high neutrophil to lymphocyte ratio (NLR) (i.e. a high baseline NLR). A high NLR is defined as a value equal to or greater than the median value. In some aspects the patient having metastatic castration-resistant prostate cancer has an NLR of 3.38 or greater. In some aspects the patient having metastatic castration-resistant prostate cancer has an NLR of 5.37 or greater. In some aspects the patient having metastatic castration-resistant prostate cancer has an NLR of from 3.38 to 101.3, for example from 5.37 to 101.3.

In some aspects, the patient having metastatic castration-resistant prostate cancer has low hemoglobin, high NLR (high baseline NLR) and a high PSA value (high PSA value at baseline). A low hemoglobin value is typically defined as lower than the median hemoglobin level. A low hemoglobin level may be lower than 121 g/L, e.g. from 10 g/dL to 121 g/L (i.e. 100 to 121 g/L). A high NLR is typically defined as a value equal to or greater than the median value. A high NLR may be an NLR of 3.38 or greater, for example from 3.38 to 101.3. A high PSA may be defined as equal to or greater than the median value. A high PSA level may be a PSA of 62.0 ng/mL, for example from 62.0 to 15000 ng/mL. Thus, in some aspects the patient having metastatic castration-resistant prostate cancer has a hemoglobin level of lower than 121 g/L ; an NLR (at baseline) of 3.38 or greater and a PSA value (at baseline) of 62.0 ng/mL or greater. In some aspects the patient having metastatic castration-resistant prostate cancer has a hemoglobin level of from 10 g/dL to 121 g/L ; an NLR (at baseline) of from 3.38 to 101.3 and a PSA value (at baseline) of from 62.0 to 15000 ng/mL.

Patients having high NLR, in particular patients having low hemoglobin, high NLR (high baseline NLR) and a high PSA value (high PSA value at baseline) are a challenging group of patients to successfully treat. The treatment described herein provided even such challenging group of patients with improved overall survival.

In some aspects, the patient having metastatic castration-resistant prostate cancer has PSA progression only, or imaging-based progression (with or without PSA progression and typically with no pain, i.e. a pain score of 0 or 1 and analgesic level of 0 or 1; wherein pain progression is determined using the Brief Pain Inventory-Short Form and analgesic consumption is determined by the World Health Organization's analgesic ladder). In certain aspects, the patient has PSA progression only. In these aspects, the patient does not have significant pain progression prior to administration with cabazitaxel (i.e. pain is at level 0 or 1). It has been found that the treatment described herein is beneficial in patients having relatively low levels of disease progression (e.g. PSA only or imaging-based progression with or without PSA progression).

Reference to the patient characteristics in the aspects described above refers to the characteristics of the patient at the point of initiating cabazitaxel therapy. Where the patient characteristic is disease progression, this is the level of disease progression at the point of initiating cabazitaxel therapy, i.e. after docetaxel and an AR-targeted agent.

### BRIEF DESCRIPTION OF THE DRAWINGS

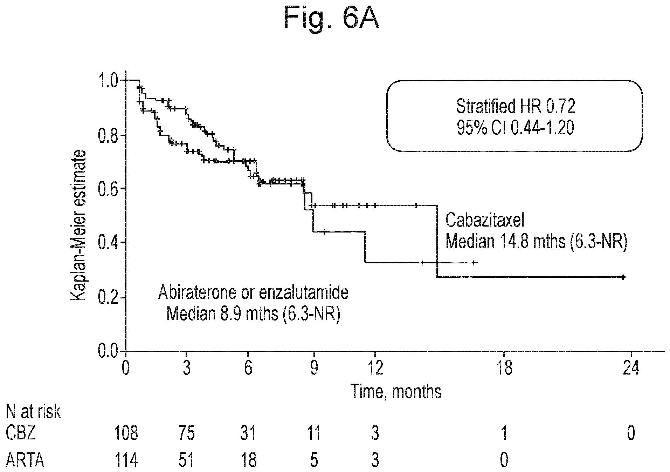

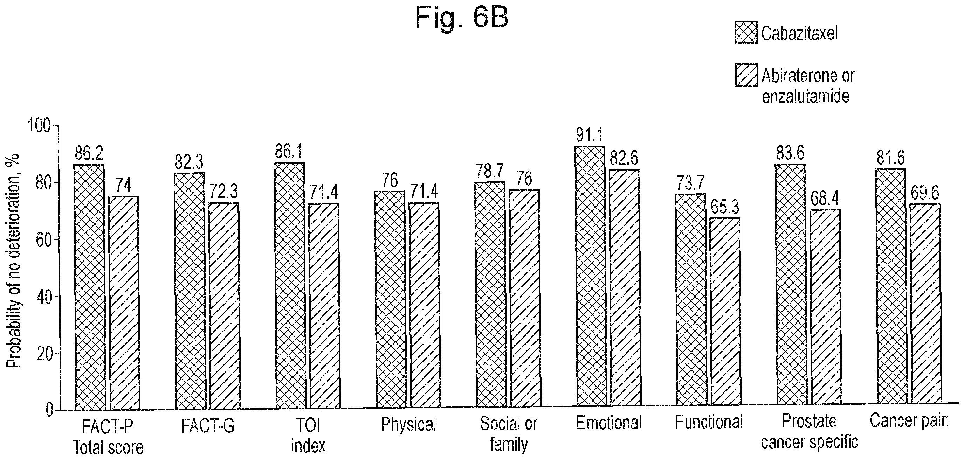

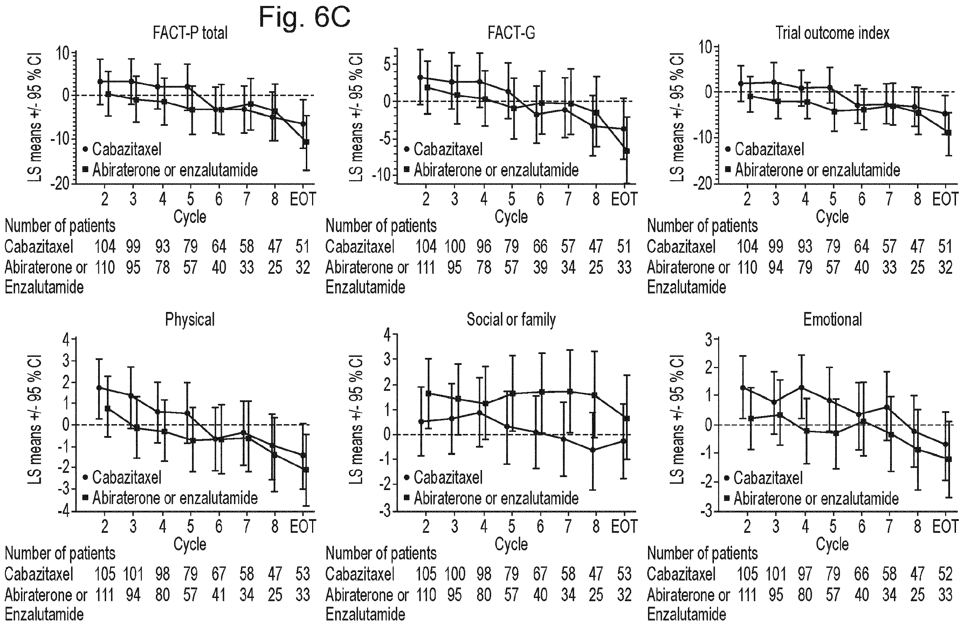

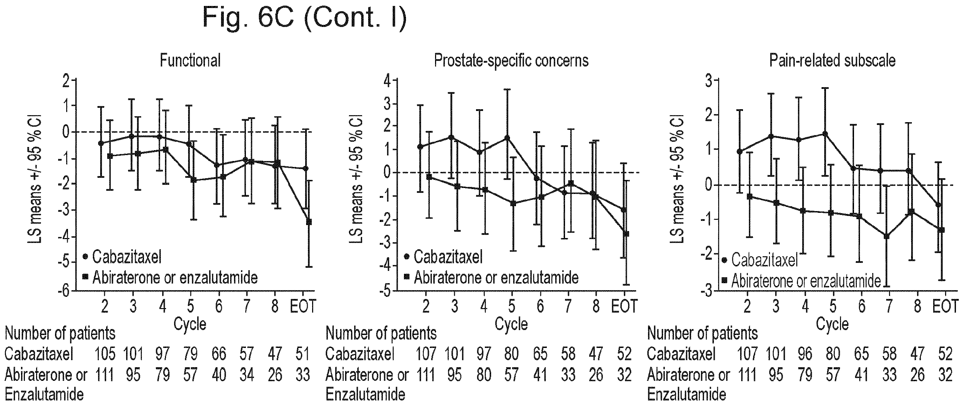

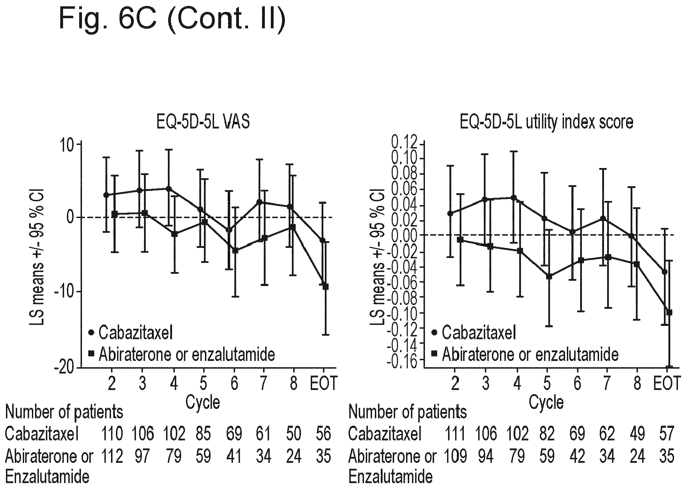

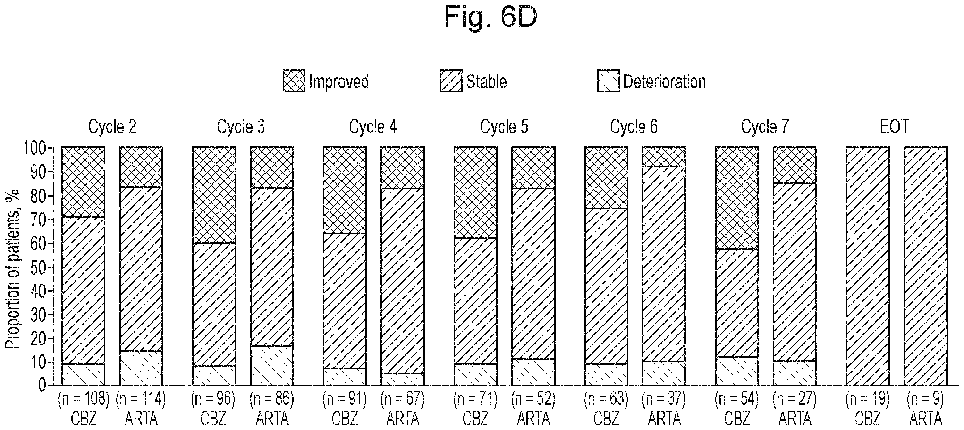

**Figure 1****.** (A) Kaplan-Meier estimates for radiographic progression-free survival and (B) corresponding subgroup analyses according the study performed in Example 1.
**Figure 2****.** Kaplan-Meier estimates for (A) overall survival and (B) progression-free survival according the study performed in Example 1.
**Figure 3****.** Kaplan-Meier estimates for time to symptomatic skeletal event according the study performed in Example 1.
**Figure 4****.** Kaplan-Meier estimates for radiographic progression-free survival in patients receiving (A) enzalutamide after prior docetaxel and abiraterone acetate and (B) abiraterone acetate after prior docetaxel and enzalutamide
**Figure 5****.** Pain response and deterioration, and time to SSE
   A-Pain response and deterioration *Pain response: decrease ≥ 30% from baseline in average BPI-SF pain intensity score at two consecutive evaluations ≥ 3 weeks apart without an increase in analgesic usage score. †Pain deterioration: increase of ≥ 30% from baseline in BPI-SF pain intensity score (item 3) observed at two consecutive evaluations ≥ 3 weeks apart without a decrease in analgesic usage score* or an increase in analgesic usage score of ≥ 30%.
   B- Time to symptomatic skeletal events (SSE): Symptomatic skeletal event: occurrence of new symptomatic pathologic fracture or use of external beam radiation therapy to relieve bone pain or occurrence of spinal cord compression or tumor-related orthopedic surgical intervention
**Figure 6****:** FACT-P total score
   A- Time to total FACT-P score deterioration
   B- FACT-P: Probability of survival without definitive deterioration at 3 months
   C- Adjusted mean (95% CI) change from baseline in (A) FACT-P total score; (B) FACT-G total score; (C) Physical well-being; (D) Social or family well-being; (E) Emotional well-being; (F) Functional well-being; (G) Prostate-specific concerns; (H) Prostate-specific concerns-pain; (I) Trial outcome index; (J) EQ-5D-5L VAS; (H) EQ-5D-5L utility index score. Only results where n>20% are shown.
   D- Percentage of patients with improvement, maintenance, or deterioration of FACT-P pain-related subscale
      Improvement in pain-related subscale score is defined as a ≥2-point increase from baseline observed at two consecutive evaluations at least 3 weeks apart. Deterioration in pain-related subscale score is defined as a ≥2-point decrease from baseline observed at two consecutive evaluations at least 3 weeks apart. A maintained pain-related subscale score means neither an improvement nor deterioration was observed.
      Abbreviations: ARTA, abiraterone; CBZ, cabazitaxel; EOT, end of treatment.
**Figure 7****:**
   A- Proportion of patients with improvements in pain/discomfort from baseline using EQ-5D-5L (CBZ: cabazitaxel; ARTA: alternative androgen-signaling-targeted inhibitor; EOT, end of treatment).
   B- Percentage of patients with improvement, maintenance, or deterioration of EQ-5D-5L pain discomfort score
      Improvement in the EQ-5D-5L pain/discomfort subscale score is defined as a ≥2-point increase from baseline observed at two consecutive evaluations at least 3 weeks apart. Deterioration in the EQ-5D-5L pain/discomfort subscale score is defined as a ≥2-point decrease from baseline observed at two consecutive evaluations at least 3 weeks apart. A maintained EQ-5D-5L pain/discomfort subscale score means neither an improvement nor deterioration was observed.

### Definitions and Abbreviations

As used herein, the following abbreviations, unless otherwise indicated, shall be understood to have the following meanings:
- AR: androgen receptor
- CI: confidence interval
- ECOG: Eastern Cooperative Oncology Group
- EOT: End of Treatment
- FACT-P: Functional Assessment of Cancer Therapy-Prostate questionnaire
- HRQL: health-related quality of life
- mCRPC: metastatic Castration Resistant Prostate Cancer
- OS: Overall survival
- PFS: Progression Free Survival
- PSA: prostate-specific antigen

### DEFINITIONS

As used herein, the following terms, unless otherwise indicated, shall be understood to have the following meanings:
Imaging-based progression-free survival (rPFS): defined as the time from randomization until the occurrence of one of the following events: objective tumor progression (Response Evaluation Criteria in Solid Tumors, version 1.1), progression of bone lesions (Prostate Cancer Working Group 2) or death due to any cause.

Overall survival (OS): defined as the time from the date of randomization to the date of death due to any cause.

"A method of increasing survival" as used herein means a method that prolongs the life of a patient as compared to treatment with an alternative AR targeted agent.

Progression-free survival (PFS) defined as the time (e.g. the time from randomization) until the occurrence of objective tumor progression (using Response Evaluation Criteria in Solid Tumors, version 1.1), or progression of bone lesions (using Prostate Cancer Working Group 2 criteria), or symptomatic progression (defined as the development of urinary or bowel symptoms related to prostate cancer or requiring a change of anticancer therapy), or pain progression, or death due to any cause.

"A method of increasing progression free survival" as used herein means a method that increases PFS as compared to treatment with an alternative AR targeted agent.

As used herein, "patient" means a human.

PSA response: defined as a decline of serum PSA from baseline of ≥50% with confirmation from a second PSA value measured at least 3 weeks apart. Best PSA response was defined using the Prostate Cancer Working Group criteria 2 definition.

Tumor response (TR): was measured using Response Evaluation Criteria in Solid Tumors, version 1.1 in patients with measurable disease.

Pain response (PR): was measured using the Brief Pain Inventory-Short Form for pain intensity score and defined as a decrease by >30% from baseline in the Brief Pain Inventory-Short Form pain intensity item scores observed at two consecutive evaluations ≥3 weeks apart without an increase in analgesic usage score.

Time to symptomatic skeletal event rate: is defined as the time interval between the date of randomization and the date of the occurrence of the first event defining a symptomatic skeletal event (occurrence of a new symptomatic fracture, use of radiation therapy to relieve bone pain, occurrence of spinal cord compression or cancer-related orthopedic surgical intervention).

Patients who failed a prior androgen signaling targeted inhibitor are defined as patients who have disease progression within 12 months of androgen signaling targeted inhibitor (≤12 months), (either before or after docetaxel). Disease progression within 12 months of androgen signaling targeted inhibitor as used herein means within 12 months of initiating therapy with the androgen signaling targeted inhibitor, as is the normal meaning of the term in the art. Typically, where docetaxel is administered prior to androgen signaling targeted inhibitor , cabazitaxel is administered within 12 months of the first administration of androgen signaling targeted inhibitor.

Patients who have disease progression after cabazitaxel therapy are typically patients who have disease progression within 12 months of treatment with cabazitaxel (≤12 months).

Disease progression (either after AR-targeted agent therapy or after cabazitaxel therapy) is defined herein as the occurrence of one of the following events: objective tumor progression (Response Evaluation Criteria in Solid Tumors, version 1.1) or progression of bone lesions (Prostate Cancer Working Group 2).

A high value of a defined marker (e.g. neutrophil level or NLR) is defined as a value equal to or above the median level. A low value of a marker is defined as a value below the median value.

As used herein, the wording "a compound for use ..." , for example, shall be understood as being equivalent to the wording "use of a compound for ..." or "use of a compound for the preparation of a medicament for use in ..."

The terms "androgen-signaling-targeted inhibitor", "AR-targeted agent", "AR targeted therapy", and "AR pathway inhibitor", as used herein, are synonymous terms that refer to therapeutic agents that are used to treat prostate cancer by targeting the androgen receptor signaling axis. Particular androgen signaling targeted inhibitors are abiraterone acetate and enzalutamide.

An "alternative AR-targeted agent" as used herein is a different AR-targeted agent than the "prior AR-targeted agent," "prior androgen signaling targeted inhibitor," "alternative androgen signaling targeted inhibitor," or "previous alternative AR pathway inhibitor" that a patient previously received; it may also be called ARTA. In some embodiments, the prior androgen signaling targeted inhibitor is abiraterone acetate, and the alternative androgen signaling targeted inhibitor is enzalutamide. In other embodiments, the prior AR-targeted agent is enzalutamide, and the alternative AR-targeted agent is abiraterone acetate.

Abiraterone acetate is a CYP17 inhibitor indicated with prednisone for the treatment with mCRPC and metastatic high-risk castration-sensitive prostate cancer. Abiraterone acetate is known chemically as (3β)-17-(3-pyridinyl) androsta-5,16-dien-3-yl acetate. Abiraterone acetate may be administered to a patient at a daily dose of 1000 mg, for example.

Enzalutamide is an androgen signaling targeted inhibitor indicated for the treatment of patients with castration resistant prostate cancer. The chemical name of enzaltamide is 4-{3-[4-cyano-3-(trifluoromethyl)phenyl]-5,5-dimethyl-4-oxo-2-sulfanylideneimidazolidin-1-yl}-2-fluoro-*N-*methylbenzamide. Enxalutamide may be administered to a patient at a daily dose of 160 mg, for example.

**Cabazitaxel** belongs to the taxoid family and has the formula:

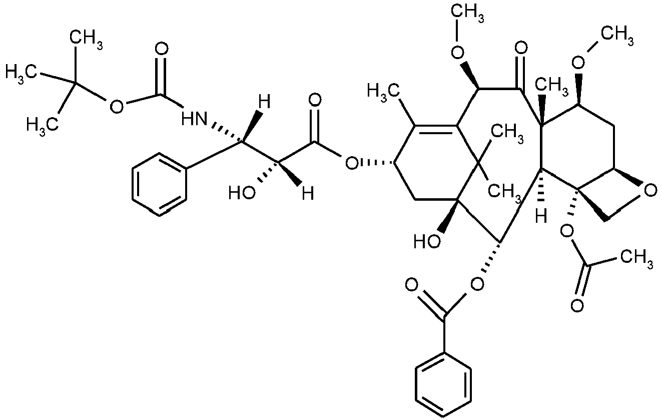

The chemical name of cabazitaxel is 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate. Cabazitaxel is synonymously known as (2α, 5β, 7β, 10β, 13α)-4-acetoxy-13-({(2R,3S)-3-[(tertbutoxycarbonyl)amino]-2-hydroxy-3-phenylpropanoyl}oxy)-1-hydroxy-7,10-dimethoxy-9-oxo-5,20-epoxytax-11-en-2-yl benzoate.

This compound and a preparative method thereof is described in WO 96/30355, EP 0 817 779 B1 and US 5 847 170, which are hereby incorporated herein by reference. Cabazitaxel may be administered in base form (cf. above formula), or in the form of a hydrate. It may also be a solvate, i.e. a molecular complex characterized by the incorporation of the crystallization solvent into the crystal of the molecule of the active principle (see in this respect page 1276 of J. Pharm. Sci. 1975, 64(8), 1269-1288). In particular, it may be an acetone solvate, and, more particularly, may be the solvate described in WO 2005/028462. It may be an acetone solvate of cabazitaxel containing between 5% and 8% and preferably between 5% and 7% by weight of acetone (% means content of acetone/content of acetone+cabazitaxel × 100). An average value of the acetone content is 7%, which approximately represents the acetone stoichiometry, which is 6.5% for a solvate containing one molecule of acetone. The procedure described below allows the preparation of an acetone solvate of cabazitaxel:
940 ml of purified water are added at 20±5°C (room temperature) to a solution of 207 g of 4α-acetoxy-2α-benzoyloxy-5β,20-epoxy-1β-hydroxy-7β,10β-dimethoxy-9-oxo-11-taxen-13α-yl (2R,3S)-3-*tert*-butoxycarbonylamino-2-hydroxy-3-phenylpropionate at about 92% by weight in about 2 litres of acetone, followed by seeding with a suspension of 2 g of 4a-acetoxy-2a-benzoyloxy-5β,20-epoxy-1β-hydroxy-7β,10β-dimethoxy-9-oxo-11-taxen-13α-yl (2R,3S)-3-*tert-*butoxycarbonylamino-2-hydroxy-3-phenylpropionate isolated from acetone/water in a mixture of 20 ml of water and 20 ml of acetone. The resulting mixture is stirred for about 10 to 22 hours, and 1.5 litres of purified water are added over 4 to 5 hours. This mixture is stirred for 60 to 90 minutes, and the suspension is then filtered under reduced pressure. The cake is washed on the filter with a solution prepared from 450 ml of acetone and 550 ml of purified water, and then oven-dried at 55°C under reduced pressure (0.7 kPa) for 4 hours. 197 g of 4a-acetoxy-2a-benzoyloxy-5β,20-epoxy-1β-hydroxy-7β,10β-dimethoxy-9-oxo-11-taxen-13α-yl (2R,3S)-3-*tert-*butoxycarbonylamino-2-hydroxy-3-phenylpropionate acetone containing 0.1% water and 7.2% acetone (theoretical amount: 6.5% for a stoichiometric solvate) are obtained.

Cabazitaxel may be administered parenterally, such as via intravenous administration. A galenical form of cabazitaxel suitable for administration by intravenous infusion is that in which the cabazitaxel is dissolved in water in the presence of excipients chosen from surfactants, cosolvents, glucose or sodium chloride, etc. For example, a galenical form of cabazitaxel may be prepared by diluting a premix solution of cabazitaxel contained in a sterile vial (80 mg of cabazitaxel + 2 ml of solvent + Polysorbate 80) with a sterile vial containing a solution of 6 ml of water and ethanol (13% by weight of 95% ethanol) in order to obtain 8 ml of a solution ready to be rediluted in a perfusion bag. The concentration of cabazitaxel in this ready-to-redilute solution is about 10 mg/ml. The perfusion is then prepared by injecting the appropriate amount of this ready-to-redilute solution into the perfusion bag containing water and glucose (about 5%) or sodium chloride (about 0.9%).

Cabazitaxel may be administered in combination with a corticoid, such as prednisone or prednisolone, as two distinct pharmaceutical preparations.

In some embodiments, the effective amount of cabazitaxel produces at least one therapeutic effect selected from the group consisting of an increase in overall survival and an increase in progression free survival.

Cabazitaxel may be administered in combination with a medication to prevent or control nausea and vomiting or to prevent or control hypersensitivity to the cabazitaxel treatment. In some aspects, a patient is pre-medicated with the medication, for example, at least 30 minutes prior to administering each dose of cabazitaxel.

Severe hypersensitivity reactions to cabazitaxel can occur and may include generalized rash/erythema, hypotension and bronchospasm. Patients should be observed closely for hypersensitivity reactions, especially during the first and second infusions. Hypersensitivity reactions may occur within a few minutes following the initiation of the infusion of cabazitaxel, thus facilities and equipment for the treatment of hypotension and bronchospasm should be available. If severe hypersensitivity reaction occurs, cabazitaxel infusion should be immediately discontinued and appropriate therapy should be administered. Examples of medications which may be used to prevent hypersensitivity to the cabazitaxel treatment include antihistamines, such as dexchloropheniramine (for example 5 mg), and diphenhydramine (for example 25 mg) or equivalent antihistamines; and corticosteroids, such as dexamethasone (for example 8 mg) or an equivalent steroid.

Nevertheless, cabazitaxel should not be given to and may be contraindicated in patients who have a history of severe hypersensitivity reactions to cabazitaxel. Depending on the formulation administered, cabazitaxel may also be contraindicated in patients who have a history of hypersensitivity reactions to other drugs formulated with polysorbate 80.

Gastrointestinal symptoms, such as, for example nausea, vomiting, and diarrhea, may occur with the treatment of cabazitaxel. Mortality related to diarrhea and electrolyte imbalance has been reported. Therefore, patients may also be rehydrated and treated with anti-diarrheal or anti-emetic medications as needed. Treatment delay or dosage reduction may be necessary if patients experience Grade ≥ 3 diarrhea.

Accordingly, in some embodiments, the methods provided herein include administering a medication to prevent hypersensitivity or a medication to prevent or control nausea and vomiting in combination with cabazitaxel.

Examples of medications which may be used to prevent or control nausea and vomiting include histamine H₂ antagonists and antiemetics, such as ondansetron, granisetron and dolesetron.

A possible side effect of the treatment with cabazitaxel is neutropenia, which is characterized by a reduced number of neutrophils. Unfortunately, a number of neutropenia deaths have been reported. Therefore, frequent blood counts should be obtained or performed to monitor for neutropenia. If neutropenia occurs, cabazitaxel treatment may be discontinued, and restarted when neutrophil counts recover to a level of >1,500 /mm³. Cabazitaxel should not be given to a patient with a neutrophil count ≤ 1,500 cells/mm³.

Accordingly, in some embodiments, the methods provided herein include monitoring blood counts in the patient, and measuring neutrophil levels. In one aspect, the method further comprises discontinuing cabazitaxel treatment if neutropenia occurs, and optionally restarting cabazitaxel treatment when neutrophil counts recover to a level of >1,500 /mm³. In one aspect, the monitoring comprises taking a blood sample from the patient.
Determining neutrophil counts can be performed according to procedures well known to those skilled in the art.

In some embodiments, the methods provided herein include administering cabazitaxel in combination with an agent useful for treating neutropenia. Such a neutropenia treatment agent is, for example, a hematopoietic growth factor which regulates the production and function of neutrophils such as a human granulocyte colony stimulating factor, (G-CSF). In a particular aspect, the neutropenia is complicated neutropenia. Complicated neutropenia includes febrile neutropenia, prolonged neutropenia, or neutropenic infection. In some embodiments, the neutropenia treatment agent is administered prior to the administration of cabazitaxel.

In a particular aspect, primary prophylaxis with G-CSF should be considered in patients with high-risk clinical features (age > 65 years, poor performance status, previous episodes of febrile neutropenia, extensive prior radiation ports, poor nutritional status, or other serious comorbidities) that predispose them to increased complications from prolonged neutropenia. Therapeutic use of G-CSF and secondary prophylaxis should be considered in all patients considered to be at increased risk for neutropenia complications.

In another aspect, the monitoring of complete blood counts is performed on a weekly basis during cycle 1 and before each treatment cycle thereafter so that the dose can be adjusted, if needed. Therefore, another aspect for reducing the risk of neutropenia complications comprises monitoring blood counts in the patient and adjusting the dose of cabazitaxel.

Cases of renal failure should be identified and managed aggressively, accordingly to procedures known to those skilled in the art. Renal failure may be associated with sepsis, dehydration, or obstructive uropathy. Furthermore, impaired hepatic function (e.g., total bilirubin ≥ ULN, or AST and/or ALT ≥ 1.5 x ULN) may increase cabazitaxel concentrations, and cabazitaxel should not be given to patients with hepatic impairment.

Cabazitaxel may cause fetal harm when administered to a pregnant woman.

Cabazitaxel is primarily metabolized through CYP3A. Concomitant administration of strong CYP3A inhibitors (for example, ketoconazole, itraconazole, clarithromycin, atazanavir, indinavir, nefazodone, nelfinavir, ritonavir, saquinavir, telithromycin, voriconazole) may increase cabazitaxel concentrations. Therefore co-administration of cabazitaxel with strong CYP3A inhibitors should be avoided. Caution should be exercised with concomitant use of moderate CYP3A inhibitors. One aspect of the invention is a method of treating a patient for prostate cancer comprising determining whether the patient is undergoing treatment with a CYP3A inhibitor, discontinuing treatment with a CYP3A inhibitor, and then administering cabazitaxel to the patient.

Concomitant administration of strong CYP3A inducer (e.g., phenytoin, carbamazepine, rifampin, rifabutin, rifapentin, phenobarbital) may decrease cabazitaxel concentrations. Therefore co-administration of cabazitaxel with strong CYP3A inducers should be avoided. Therefore in some embodiments, the methods provided herein include determining whether the patient is undergoing treatment with a CYP3A inducer, discontinuing treatment with a CYP3A inducer, and administering cabazitaxel to the patient.

In addition, patients should also refrain from taking St. John's Wort.

For the purpose of clarity and a concise description, features are described herein as part of the same or separate aspects and preferred embodiments thereof, however, it will be appreciated that the scope of the invention may include embodiments having combinations of all or some of the features described.

The invention will now be illustrated by the following examples, which are provided by way of illustration and not of limitation and it will be understood that many variations in the methods described and the amounts indicated can be made without departing from the spirit of the invention and the scope of the appended claims.

### EXAMPLES

A clinical study was performed wherein patients received cabazitaxel versus an Androgen Receptor (AR)-targeted agent (abiraterone acetate or enzalutamide) in metastatic castration-resistant prostate cancer (mCRPC) patients previously treated with docetaxel and who failed a prior androgen signaling pathway inhibitor within 12 months.

For patients in Arm A, cabazitaxel 25 mg/m² was administered intravenously over 1 hour every 3 weeks along with oral prednisone 10 mg given daily. Primary prophylactic granulocyte-colony stimulating factor (G-CSF) was required at each cycle for all patients in the cabazitaxel arm For patients in Arm B, abiraterone acetate was administered orally at 1000 mg once daily along with oral prednisone 5 mg given twice daily or enzalutamide was administered orally at 160 mg once daily continuously. Abiraterone acetate was administered to patients who had received enzalutamide prior to study entry. Enzalutamide was administered to patients who received prior abiraterone acetate.

Each patient was treated until imaging-based disease progression, unacceptable toxicity, or patient's refusal of further study treatment. All patients were followed when on study treatment and after completion of study treatment during follow-up period until death, the study cut-off date, or withdrawal of patients' consent whichever came first.

The sample size of 234 patients in 2 arms (117 patients per arm) was anticipated to be needed to reach the targeted number of patients with event. A total of 196 patients with event was needed to achieve 80% power to demonstrate imaging-based PFS superiority of cabazitaxel over androgen signaling targeted inhibitors when considering a HR of 0.67 that was considered as the smallest effect of clinical interest between cabazitaxel and an AR-targeted agent (abiraterone or enzalutamide) by 2 sided log rank test at 0.05 type I error rate.

### Patients

Eligible patients had histologically confirmed prostate cancer, castrate levels of serum testosterone (<0.5ng/mL), were previously treated with ≥3 cycles docetaxel, had disease progression (Response Evaluation Criteria in Solid Tumors, version1.1) and/or appearance of ≥2 new bone lesions and/or rising prostate-specific antigen (PSA) using Prostate Cancer Working Group 2 criteria, ≤12 months of treatment with a previous androgen signaling targeted inhibitor (abiraterone acetate or enzalutamide, before or after docetaxel) (Scher et al., J Clin Oncol. 2008;26(7):1148-1159). Abiraterone acetate or docetaxel in the metastatic hormone-sensitive setting was allowed. Type of progression at enrollment was classified as: PSA progression only, imaging-based progression (+/- PSA progression; no pain [0 to 1] progression using the Brief Pain Inventory-Short Form and analgesic level 0 to 1) or pain progression (Brief Pain Inventory-Short Form >1 and/or analgesic level 2 to 3; +/- PSA or imaging-based progression).

### Eligibility criteria:

1. Histologically confirmed prostate cancer
2. Metastatic disease
3. Castrate levels of serum testosterone (<0.5 ng/mL); Treatment with luteinizing hormone-releasing hormone agonists or antagonists had to be continued during the study
4. Disease progression (measurable using Response Evaluation Criteria in Solid Tumors, version 1.1 and/or appearance of ≥2 new bone lesions and/or rising prostate-specific antigen (PSA) using Prostate Cancer Working Group 2 criteria,
5. Having received prior docetaxel for at least three cycles; docetaxel in newly diagnosed metastatic setting and docetaxel rechallenge allowed
6. Progression within 12 months of treatment with a prior androgen signaling targeted inhibitor (abiraterone acetate or enzalutamide, before or after docetaxel); abiraterone acetate in metastatic hormone- sensitive setting allowed
7. Eastern Cooperative Oncology Group performance status ≤1 (patients with an Eastern Cooperative Oncology Group performance status of 2 were allowed if related to prostate cancer)
8. PSA value of at least 2ng/mL at study entry
9. Prior androgen signaling targeted inhibitor must be stopped at least 2 weeks before study treatment
10. Signed informed consent

### Exclusion criteria:

1. Prior chemotherapy other than docetaxel for prostate cancer, except estramustine and except adjuvant/neoadjuvant treatment completed >3 years ago
2. Less than 28 days elapsed from prior treatment with chemotherapy, immunotherapy, radiotherapy or surgery to the time of randomization
3. Adverse events (excluding alopecia and those listed in the specific exclusion criteria) from any prior anticancer therapy of Grade >1 (National Cancer Institute Common Terminology Criteria v4.0) at the time of randomization
4. Less than 18 years (or country's legal age of majority if the legal age is >18 years)
5. Eastern Cooperative Oncology Group performance status >2 (Eastern Cooperative Oncology Group performance status 2 must be related to prostate cancer, not to other comorbidities)
6. Prior malignancy; adequately treated basal cell or squamous cell skin or superficial (pTis, pTa, and pT1) bladder cancer are allowed, as well as any other cancer for which treatment has been completed ≥5 years ago and from which the patient has been disease-free for ≥5 years
7. Participation in another clinical trial and any concurrent treatment with any investigational drug within 30 days prior to randomization
8. Acquired immunodeficiency syndrome (AIDS-related illnesses) or known HIV disease requiring antiretroviral treatment
9. Patients with reproductive potential who do not agree, in conjunction with their partner, to use accepted and effective method of contraception during the study treatment period and up to 6 months after the last administered dose; the definition of "effective method of contraception" described hereafter: oral contraceptives, combined hormonal intravaginal, transdermal, or intra uterine device or will be based on country-specific regulatory requirements, and documented in the Informed Consent Form
10. Known allergies, hypersensitivity or intolerance to prednisone or excipients of abiraterone acetate or enzalutamide or docetaxel or polysorbate 80
11. Known history of mineralocorticoid excess or deficiency
12. History of seizure, underlying brain injury with loss of consciousness, transient ischemic attack within the past 12 months, cerebral vascular accident, brain arteriovenous malformation, brain metastases or the use of concomitant medications that may lower the seizure threshold
13. Unable to swallow a whole tablet or capsule
14. Inadequate organ and bone marrow function as evidenced by:
   a. Hemoglobin <10.0 g/dL
   b. Absolute neutrophil count <1.5 x 10⁹/L
   c. Platelet count <100 x 10⁹/L
   d. Aspartate transferase and/or alanine transferase >1.5 x ULN;
   e. Total bilirubin >1.0 x ULN
   f. Potassium <3.5 mmol/L
   g.Child-Pugh Class C
15. Contraindications to the use of corticosteroid treatment
16. Symptomatic peripheral neuropathy Grade ≥2 (National Cancer Institute Common Terminology Criteria v.4.0)
17. Uncontrolled severe illness or medical condition including uncontrolled diabetes mellitus, history of cardiovascular disease (uncontrolled hypertension, arterial thrombotic events in the past 6 months, congestive heart failure, severe or unstable angina pectoris, recent myocardial infraction within last 6 months or uncontrolled cardiac arrhythmia)
18. Concomitant vaccination with yellow fever vaccine

### Randomization and treatment

Eligible patients were randomized to receive either cabazitaxel or an AR pathway inhibitor (abiraterone acetate or enzalutamide; dependent on the use of a previous androgen signaling targeted inhibitor) in a 1:1 ratio. Stratification criteria were Eastern Cooperative Oncology Group performance status, a 5-point scale where higher numbers indicate greater disability (0-1 vs. 2), and time to disease progression (≤6 months vs. >6-12 months) and timing (before vs. after docetaxel) with previous alternative AR pathway inhibitors.

Cabazitaxel 25mg/m², was administered intravenously over 1 hour every 3 weeks with oral prednisone 10mg daily. Premedication included an antihistamine, glucocorticoid (dexamethasone 8mg or equivalent), and histamine 2 receptor antagonist. Antiemetic prophylaxis was administered at physician's discretion. Primary prophylactic granulocyte-colony stimulating factor was required during each cycle of cabazitaxel. Patients received either abiraterone acetate (1000mg orally once daily and oral prednisone 5mg twice daily) or enzalutamide (160mg orally once daily) continuously. A treatment cycle was 3 weeks in both arms. Abiraterone acetate was given to patients who previously received enzalutamide before study entry. Enzalutamide was given to patients who previously received abiraterone acetate.

Up to a maximum of 2 dose reductions was allowed per patient when necessary due to toxicity and according to the table below:

### Cabazitaxel Dose Reduction Levels

| | Initial dose (mg/m²) | Dose Reduction 1 | | Dose Reduction 2 |
|---|---|---|---|---|
| Cabazitaxel | 25 | 20 | → | 15 |

The study was open label. Each patient was treated until imaging-based disease progression, unacceptable toxicity, start of a subsequent treatment, or by patient request.

### End points

The primary end point for the study was imaging-based progression-free survival, defined as time from randomization until objective tumor progression (using Response Evaluation Criteria in Solid Tumors, version1.1), progression of bone lesions (using Prostate Cancer Working Group 2 criteria) or death. There was no blinded central review of imaging. Secondary end points included overall survival, progression-free survival, PSA response, tumor and pain responses, time to symptomatic skeletal events and safety.

### Pain response

Pain response was defined as a decrease ≥ 30% from baseline in BPI-SF pain intensity score with no increased analgesic use. HRQL was assessed using the Functional Assessment of Cancer Therapy-Prostate (FACT-P) questionnaire. Pain intensity was defined using item 3 of the validated Brief Pain Inventory-Short Form (BPI-SF), which rates pain at its worst in the last 24 hours on a scale of 0 (no pain) to 10 (worst imaginable pain). Analgesic consumption was reported by local investigators and coded by the World Health Organization's analgesic ladder (0 = no analgesic; 1 = non-opioid analgesics; 2 = opioid analgesics for moderate pain; 3 = opioids for severe pain). Patients were evaluable if they had received at least one dose of cabazitaxel or abiraterone or enzalutamide and had a baseline FACT-P score plus at least one subsequent FACT-P measurement. A clinically meaningful improvement or deterioration of total FACT-P score was defined as a difference of ± 10 points from baseline. Clinically meaningful changes in HRQL and pain response were confirmed at two consecutive evaluations ≥ 3 weeks apart during the on-treatment period. Survival curves were generated by Kaplan-Meier estimates.

Time to deterioration was defined on two consecutive evaluations, ≥ 3 weeks apart as follows:
- decrease of ≥ 10 points from baseline for FACT-P total score
- decrease of ≥3 points from baseline for physical, social, emotional and functional well-being
- decrease of ≥ 2 points from baseline for the pain PCS subscale
- decrease of ≥9 points from baseline for FACT-G total and FACT-P TOI subscales

FACT-P, a validated 39-item questionnaire evaluated 5 wellbeing domains: physical (PWB, 0-28), social and family (SWB, 0-28), emotional (EWB, 0-24), functional (FWB, 0-28), and prostate specific (PCS, 0-48).

Total FACT-P score (PWB+SWB+EWB +FWB+PCS; 0-156), total FACT-G score (PWB+SWB+EWB+FWB; 0-108), FACT-P trial outcome index (PWB+FWB+PCS; 0-104), and PCS-Pain were calculated. The FACT-P overall score ranges from 0 to 156, higher values indicating a higher HRQL

SSEs were defined as either the use of external beam radiation to relieve bone pain, occurrence of new symptomatic pathologic fractures, occurrence of spinal cord compression, or tumor-related orthopedic surgical intervention. SSEs were evaluated at baseline, at each visit, at the end of treatment, and every 12 weeks during follow-up. Standard imaging (bone scans and computed tomography or magnetic resonance imaging of the pelvis, abdomen, and chest) was scheduled at baseline and every 12 weeks until imaging-based disease progression. Pathologic fractures and spinal cord compression were assessed by imaging as needed throughout the study. Time to SSE was defined as time from randomization until first documented SSE.

### Patient-reported outcomes (PROs):

PROs were evaluated using the EQ-5D-5L utility index and visual analogue scale (VAS). Scores were evaluated at baseline, on Day 1 of each cycle and at the end of treatment (EOT) visit. Patients were evaluable if they had received at least one dose of CBZ or ARTA and had an EQ-5D-5L assessment at baseline and at least one subsequent assessment. A mixed-effect model repeated measures analysis of EQ-5D-5L changes from baseline was performed. EQ-5D-5L describes five dimensions of health: mobility, self-care, usual activities, pain/discomfort, and anxiety/depression. Patients rate each domain on a 1-5 Likert-type scale (1 = no problems, 2 = slight problems, 3 = moderate problems, 4 = severe problems, 5 = extreme problems)

### Statistical analysis

The study was designed to have 80% power to detect a hazard ratio of 0.67 (cabazitaxel vs. androgen signaling targeted inhibitor) for imaging-based progression, using a stratified log-rank test at two-sided alpha level of 5%. Approximately 234 patients needed to be randomized to obtain 196 events. All efficacy analyses used data obtained at this cut-off date, as specified in the protocol. If an imaging event or death did not occur during the study then imaging-based progression-free survival data were censored at last tumor assessment or at the cut-off date, whichever came first. If no valid tumor assessment was available, data were censored at the randomization date. No prior interim analysis was performed. Final analysis will occur after all patients reach the end of the study and will be descriptive.

Efficacy analysis used all randomized patients. Stratified log-rank tests were used to analyze time-to-event data. The primary analysis compared radiographic progression-free survival between the two treatment groups using a stratified log-rank test. Survival curves were generated using Kaplan-Meier estimates. Hazard ratios and associated 95% confidence intervals [CI] were estimated using a stratified Cox proportional hazards model. Stratified Cochran-Mantel-Haenszel chi-square tests were used to analyze categorical data. Descriptive statistics were used to summarize patient characteristics. The safety population (randomized patients who received at least one dose of study treatment) was used for all safety analyses.

To control for type I error due to multiple comparisons, a hierarchical testing procedure was applied for the primary and key secondary end points. Only if imaging-based progression-free survival was significantly different between two treatment groups would key secondary end points be tested in the following order: overall survival, progression-free survival, PSA response, and tumor response. Further tests were stopped once a comparison was found not statistically significant at two-sided alpha level of 0.05. Descriptive statistics for FACT-P and EQ-5D-5L scores were provided for all cycles for which the number of evaluable patients reached ≥20% of the PRO population. For qualitative parameters, proportions of patients with deterioration, no change, and improvement were provided.

The comparison of changes from baseline on PROs was analyzed using a mixed linear repeated measures model where treatment is a fixed effect variable and subject is a random effect variable. Stratification variables were included in the model as covariates as well as the interaction treatment visit. The least square means by treatment group with the corresponding 95% Cl obtained from the mixed model were presented graphically. No adjustments for multiplicity were made.

Compliance with the planned assessment schedule was calculated at baseline and for each treatment cycle as the proportion of patients still receiving study treatment at this time point. No formal imputation for missing data was carried out and reasons for missing data were not centrally recorded. FACT-P total score was evaluable when >80% of questions were answered. For individual FACT-P subscales, a score was evaluable when >50% of the questions in the subscale domain were answered. If <50% of the questions were missing in any FACT-P subscale, the subscale score could be imputed by prorated subscale scores using the following formula: Prorated subscale score = [Sum of question scores] x [N of questions in subscale] ÷ [N of questions answered].

### RESULTS

### Baseline and treatment characteristics

255 patients were randomly allocated to receive cabazitaxel (129 patients) or an AR pathway inhibitor (abiraterone acetate or enzalutamide; 126 patients), representing the intention-to-treat population. Of these, 250 patients were treated (126 and 124 with cabazitaxel and an androgen signaling targeted inhibitor, respectively). Of the 124 patients receiving an androgen signaling targeted inhibitor, 58 received abiraterone acetate and 66 received enzalutamide. Two patients in the cabazitaxel arm were lost to follow-up. Median follow-up was 9.2 months (from randomization to end of study).

Patient baseline characteristics are described in **Table 1**. The median age was 70 years, with 31% aged over 75 years. At randomization, 21 patients (8.2%) had PSA progression only, 39 patients (15.3%) had imaging-based progression and 176 (69.0%) had pain progression. Metastases were present at diagnosis in 42.7% of patients and 44.3% had a duration of response to first androgen deprivation therapy of less than 1 year.

**Table 1. Baseline characteristics of the patients**

| | **Cabazitaxel N = 129** | **androgen signaling targeted inhibitor N = 126** |
|---|---|---|
| **Baseline characteristics** | | |
| Median age, years (range) | 70.0 (46-85) | 71.0 (45-88) |
| ≥ 75 years, n (%) | 45 (34.9) | 34 (27.0) |
| Eastern Cooperative Oncology Group performance status, n (%) | | |
| 0-1 | 123 (95.3) | 119 (94.4) |
| 2 | 6 (4.7) | 7 (5.6) |
| Liver and/or lung metastases, n (%) | 21 (16.3) | 25 (19.8) |
| PSA, ng/mL | | |
| Mean (SD) | 264.4 (1352.5) | 232.9 (453.8) |
| Median (range) | 62.0 (1.1-15000.0) | 60.5 (1.5-2868.0) |
| Neutrophil to lymphocyte ratio | | |
| Mean (SD) | 5.37 (9.37) | 4.48 (4.32) |
| Median (range) | 3.38 (0.8-101.3) | 3.37 (0.9-30.9) |
| Neutrophil count (10⁹/L) | | |
| Mean (SD) | 5.0 (2.0) | 4.7 (1.7) |
| Median (range) | 4.5 (2-11) | 4.5 (2-9) |
| Hemoglobin (g/L) | | |
| Mean (SD) | 122.0 (14.1) | 121.2 (14.1) |
| Median (range) | 121.0 (91-170) | 122.0 (82-162) |
| Alkaline phosphatase (IU/L) | | |
| Mean (SD) | 226.6 (322.2) | 235.3 (306.8) |
| Median (range) | 132.5 (41-2275) | 122.0 (35-1980) |
| Lactate dehydrogenase (IU/L) | | |
| Mean (SD) | 331.0 (276.3) | 348.5 (348.3) |
| Median (range) | 248.0 (135-2753) | 251.0 (50-3374) |
| Type of progression at study entry, n (%) | | |
| PSA only | 11 (8.5) | 10 (7.9) |
| Radiologic (± PSA) | 23 (17.8) | 16 (12.7) |
| Pain (± PSA, ± radiologic) | 86 (66.7) | 90 (71.4) |
| Missing | 9 (7.0) | 10 (7.9) |

| **Disease history** | | |
|---|---|---|
| M1 disease at diagnosis, n (%) | 49 (38.0) | 60 (47.6) |
| Gleason score 8-10 at diagnosis, n (%) | 73 (56.6) | 81 (64.3) |
| Median duration of first androgen-deprivation therapy, months (range) | 13.7 (2-114) | 12.6 (3-179) |
| Duration <12 months, n (%) | 56 (43.4) | 57 (45.2) |
| Prior androgen signaling targeted inhibitors, n (%) | 56 (43.4) | 67 (53.2) |
| Abiraterone acetate | 72 (55.8) | 59 (46.8) |
| Enzalutamide | 1 (0.8) | 0 |
| Missing | 50 (38.8) | 49 (38.9) |
| Before docetaxel | 79 (61.2) | 77 (61.1) |
| After docetaxel | | |
| Prior androgen signaling targeted inhibitor initiation to progression ≤6 months, n (%) | 65 (50.4) | 62 (49.2) |
| Bisphosphonate or denosumab use at baseline, n (%) | 27 (20·9) | 46 (36·5) |

AR, androgen receptor; PSA, prostate-specific antigen, SD, standard deviation.

| | **Cabazitaxel (N = 129)** | **Abiraterone or enzalutamide (N = 126)** |
|---|---|---|
| **Pain intensity at baseline (BPI-SF, item 3)** | | |
| 0 or 1 | 36 (27.9%) | 28 (22.2%) |
| 2 or 3 | 28 (21.7%) | 32 (25.4%) |
| ≥ 4 | 56 (43.4%) | 56 (44.4%) |
| Missing | 9 (7.0%) | 10 (7.9%) |
| **Analgesics consumption for cancer pain at baseline** | | |
| No analgesics | 57 (44.2%) | 53 (42.1%) |
| Non-opioid analgesics (mild pain) | 24 (18.6%) | 30 (23.8%) |
| Opioids pain for moderate | 16 (12.4%) | 15 (11.9.%) |
| Opioids severe pain | 31 (24.0%) | 27 (21.4%) |
| Missing | 1 (0.8%) | 1 (0.8%) |
| **Baseline FACT-P scores, mean (SD)** | n = 108 | n = 114 |
| Total FACT-P score | 103.8 (22.0) | 105.4 (21.2) |
| Total FACT-G score | 73.7 (16.5) | 75.1 (16.6) |
| Prostate cancer subscale | 30.0 (7.1) | 30.1 (6.9) |
| Pain-related score | 9.8 (4.4) | 10.2 (3.9) |
| Trial outcome index | 66.5 (16.7) | 68.1 (15.7) |
| Emotional wellbeing | 16.2 (4.5) | 16.8 (4.7) |
| Functional wellbeing | 15.6 (6.1) | 16.9 (6.0) |
| Physical wellbeing | 20.9 (5.6) | 21.0 (5.3) |
| Social and family wellbeing | 21.1 (4.8) | 20.6 (5.2) |
| Baseline EQ-5D-5L scores, mean (SD) | | |
| Number of patients, n (%) | 113 (87·6) | 112 (88·9) |
| Health status score (VAS) | 65·8 (20·4) | 66·3 (18·5) |
| Number of patients, (%) | 112 (86·8) | 115 (91·3) |
| Health utility index | 0·7 (0·26) | 0-7 (0·22) |

The median duration of treatment was longer for patients receiving cabazitaxel vs. an androgen signaling targeted inhibitor (22.0 vs. 12.5 weeks) and the median number of treatment cycles received was higher for patients receiving cabazitaxel (7 vs. 4). The principal reasons for treatment discontinuation for cabazitaxel and the androgen signaling targeted inhibitor were disease progression (43.7% vs. 71.0%) or an adverse event (19.8% vs. 8.9%).

### Primary end point

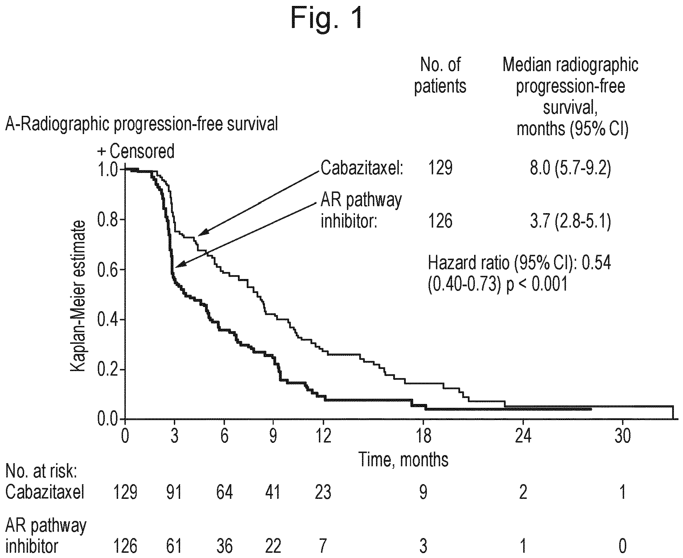

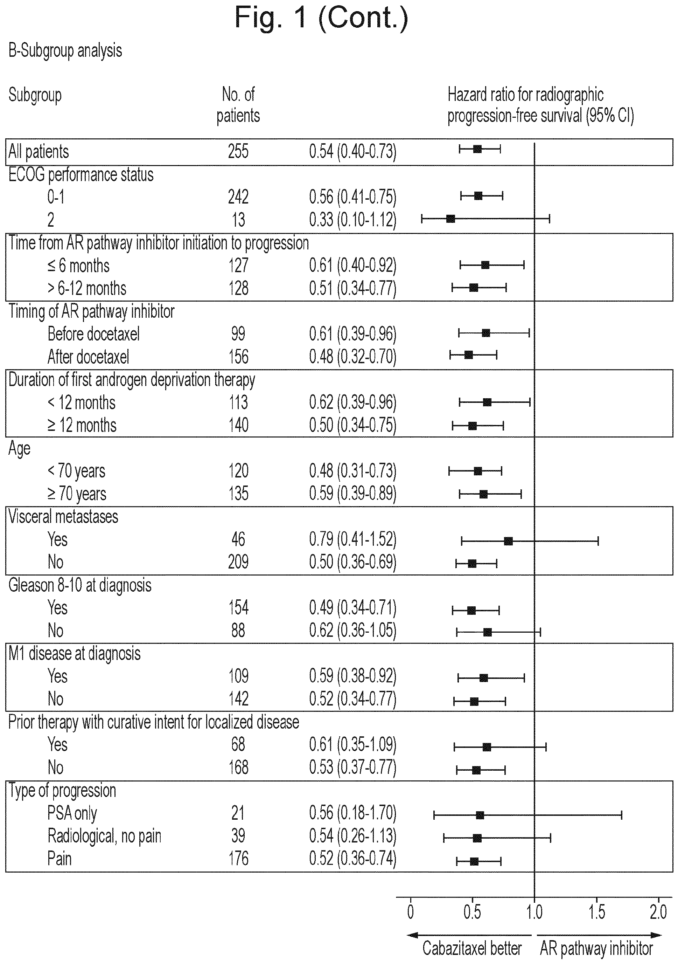

At the cut-off date, imaging-based disease progression or death from any cause were reported in 196 patients, 95 (73.6%) with cabazitaxel and 101 (80.2%) with an AR pathway inhibitor. Median imaging-based progression-free survival was 8.0 months in the cabazitaxel arm vs. 3.7 months in the androgen signaling targeted inhibitor arm (hazard ratio, 0.54; 95% Cl, 0.40 to 0.73; P<0.001) (**Figure 1**). Treatment effect on imaging-based progression-free survival was consistent through all pre-specified subgroups.

### Key secondary end points

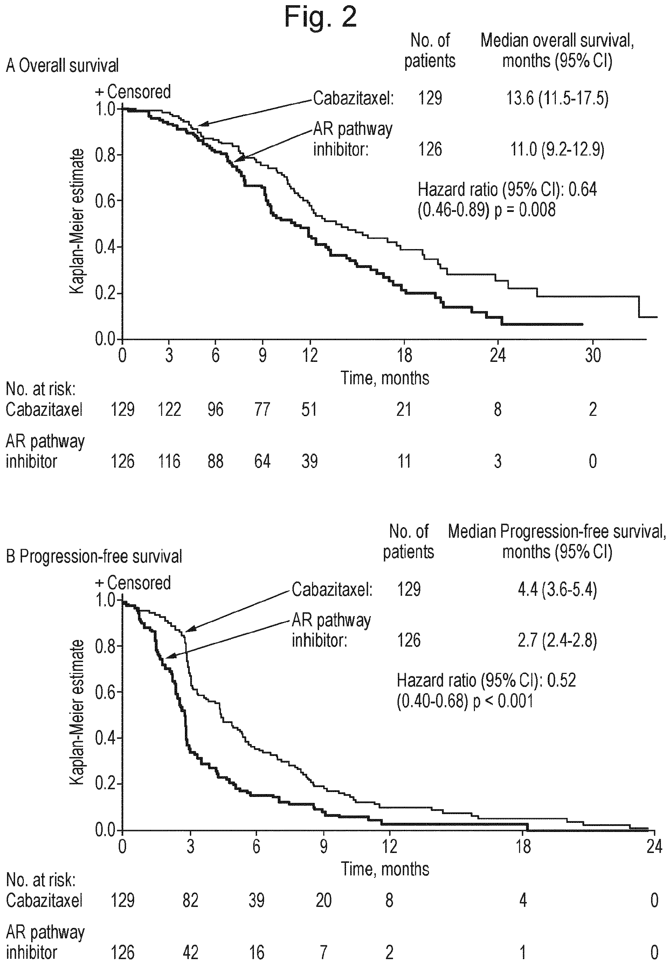

At cut-off date, 153 deaths were noted, 70 (54.3%) with cabazitaxel vs. 83 (65.9%) with an androgen signaling targeted inhibitor. Patients receiving cabazitaxel had a median overall survival of 13.6 months compared with 11.0 months for patients who received an androgen signaling targeted inhibitor (hazard ratio, 0.64; 95% Cl, 0.46 to 0.89; P=0.008) (**Figure 2A** **Table 3**).

Progression events were noted in 111 (86.0%) patients receiving cabazitaxel and 115 (91.3%) receiving an androgen signaling targeted inhibitor. Median progression-free survival was 4.4 months for patients treated with cabazitaxel vs. 2.7 months for patients treated with an androgen signaling targeted inhibitor (hazard ratio, 0.52; 95% Cl, 0.40 to 0.68; P<0.001) (**Figure 2B****; Table 3**).

OS was numerically improved for cabazitaxel vs abiraterone/enzalutamide when assessed from the time of diagnosis of metastatic disease or mCRPC, or from start of 1st or 2nd LET (life-extending therapy) (**Table 2**). In the multivariate analysis, low hemoglobin, high baseline neutrophil to lymphocyte ratio, and high PSA values at baseline were associated with worse OS. In presence of these factors, the OS benefit observed with cabazitaxel versus abiraterone/enzalutamide remained significant (HR = 0.63, 95% CI 0.42-0.94, p = 0.022).

**Table 2:**

| OS from time of | **Median OS, months** | |
|---|---|---|
| | **Cabazitaxel n = 129** | **Abiraterone/enzalutamide n = 126** |
| Metastatic disease diagnosis | 54.7 | 42.5 |
| mCRPC diagnosis | 40.9 | 31.2 |
| 1st LET | 36.4 | 30.5 |
| 2nd LET | 24.2 | 21.9 |
| 3rd LET | 13.6 | 11.0 |

Cabazitaxel numerically improved OS vs abiraterone/enzalutamide in patients with mCRPC previously treated with docetaxel and the alternative ART (abiraterone/enzalutamide), whatever the time point considered. The robustness of this OS benefit was confirmed by stratified multivariate analysis.

PSA response was evaluated in 115 and 111 patients for cabazitaxel and an androgen signaling targeted respectively. PSA reduction ≥50% from baseline, confirmed by a second value at least 3 weeks later, was observed in 35.7% of patients treated with cabazitaxel vs. 13.5% with an androgen signaling targeted inhibitor (P<0.001) (**Table 3**).

Tumor response was evaluated in 63 and 52 patients receiving cabazitaxel and an androgen signaling targeted inhibitor, respectively. Tumor response rate in patients with measurable disease at baseline was 36.5% with cabazitaxel vs. 11.5% with an androgen signaling targeted inhibitor (P=0.004) (**Table 3**).

Also, cabazitaxel doubled rPFS irrespective of age with a HR of 0.58 in patients 70 years or older and 0.47 in those aged < 70 years. Regarding overall survival, there was a 34% reduction in the risk of death with cabazitaxel vs abiraterone or enzalutamide, both in patients aged 70 years or older and those < 70 years. Other secondary endpoints, including progression-free survival, PSA response, tumor response and pain response also favored cabazitaxel vs abiraterone or enzalutamide, regardless of age.

Regarding safety, grade ≥ 3 adverse events occurred with a higher frequency in patients ≥ 70 vs < 70 years of age. Grade ≥ 3 asthenia, diarrhea and febrile neutropenia adverse events occurred more frequently in patients ≥ 70 years of age receiving cabazitaxel. Grade ≥ 3 cardiac disorders, infection, renal adverse events occurred more frequently in patients ≥ 70 years of age receiving abiraterone or enzalutamide.

### Other efficacy outcomes

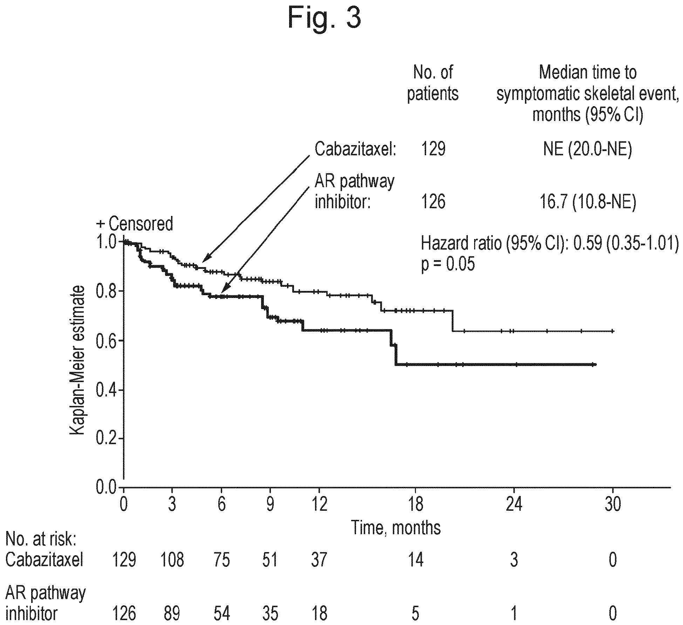

Pain response was evaluable in 111 patients treated with cabazitaxel and 109 treated with an androgen signaling targeted inhibitor. Confirmed pain response was achieved by 45.0% of patients with cabazitaxel vs. 19.3% with an androgen signaling targeted inhibitor (P<0.001) (**Table 3**). The median time to pain progression was not reached with cabazitaxel and 8·5 months with abiraterone or enzalutamide (HR: 0·55; 95% CI: 0·32-0·97; p=0·0348).
Skeletal events occurred in 24 patients (18.6%) receiving cabazitaxel and 35 patients (27.8%) receiving an androgen signaling targeted inhibitor. Median time to a symptomatic skeletal event was not reached for cabazitaxel vs. 16.7 months for the androgen signaling targeted (hazard ratio, 0.59; 95% Cl, 0.35 to 1.01; P=0.05) (**Figure 3****; Table 3**). For cabazitaxel vs. androgen signaling targeted inhibitors, 28.8% vs 51.4% of patients were estimated to have a symptomatic skeletal event at 18 months.

**Table 3:**

| | | | | |
|---|---|---|---|---|
| ***Primary and secondary end points*** | | | | |

| | **Cabazitaxel N = 129** | **androgen signaling targeted inhibitor N = 126** | **Hazard ratio (95% CI)** | **P-value** |
|---|---|---|---|---|
| **Primary end point** | | | | |
| **Imaging-based progression-free survival** | | | | |
| Median, months | 8.0 | 3.7 | 0.54 (0.40 to 0.73) | P < 0.001 |

| **Key secondary end points** | | | | |
|---|---|---|---|---|
| **Overall survival** | | | | |
| Median, months | 13.6 | 11.0 | 0.64 (0.46 to 0.89) | P = 0.008 |

| **Progression-free survival** | | | | |
|---|---|---|---|---|
| Median, months | 4.4 | 2.7 | 0.52 (0.40 to 0.68) | P < 0.001 |

| **PSA response** | | | | |
|---|---|---|---|---|
| No. of evaluable patients | 115 | 111 | | |
| Confirmed PSA reduction ≥ 50% from baseline | 41 (35.7) | 15 (13.5) | - | P < 0.001 |

| **Tumor response** | | | | |
|---|---|---|---|---|
| No. of evaluable patients | 63 | 52 | | |
| Type of response, n (%) | | | - | - |
| Partial | 23 (36.5) | 6 (11.5) | | |
| Complete | 0 | 0 | | |
| Stable disease | 23 (36.5) | 18 (34.6) | | |
| Progressive | 15 (23.8) | 28 (53.8) | | |
| disease | | | | |
| Not evaluable | 2 (3.2) | 0 | | |
| Overall response rate, n (%) | 23 (36.5) | 6 (11.5) | - | P = 0.004 |

| **Other efficacy outcomes** | | | | |
|---|---|---|---|---|
| **Pain response** | | | | |
| No. of evaluable patients | 111 | 109 | | |
| Patients with a confirmed pain response, n (%) | 50 (45.0) | 21 (19.3) | - | P < 0.001 |

| **Time to symptomatic skeletal events** | | | | |
|---|---|---|---|---|
| *Median, months | NR | 16.7 | 0.59 (0.35 to 1.01) | P = 0.05 |

| | | | | |
|---|---|---|---|---|
| AR, androgen receptor; CI, confidence interval; PSA, prostate-specific antigen. | | | | |

SSEs were observed in 24/129 patients (18.6%) in the cabazitaxel arm and 35/126 patients (27.8%) in the abiraterone or enzalutamide arm.Despite a lower use of denosumab or bisphosphonates in patients treated with cabazitaxel (20.9%) versus abiraterone or enzalutamide (36.5%), the median time to SSEs was not reached with cabazitaxel and was 16.7 months with abiraterone or enzalutamide (HR: 0·59, 95% CI: 0·35-1·01, p=0·0499). The Kaplan-Meier probability of not having SSEs with cabazitaxel versus abiraterone or enzalutamide was 92·6% versus 81·4% at 3 months and 71·2% versus 48·6% at 18 months.

**Table 4: Distribution of first symptomatic skeletal events and probability of not having symptomatic skeletal events in the intention to treat population**

| | **Cabazitaxel (n=129)** | **Abiraterone or enzalutamide (n=126)** |
|---|---|---|
| Any symptomatic skeletal event, n (%) | 24 (186·) | 35 (27·8) |
| Radiation to relieve bone pain, n (%) | 14 (10·9) | 23 (18·3) |
| Spinal cord compression, n (%) | 4 (3·1) | 4 (3·2) |
| New symptomatic pathologic fracture, n (%) | 6 (4·7) | 8 (6·3) |
| Surgery to the bone, n (%) | 0 | 0 |
| Kaplan-Meier probability of not having symptomatic skeletal events, % (95% CI) | | |
| - At 3 months | 92·6 (88·0-97·3) | 81·4 (74·4-88·5) |
| - At 6 months | 85·8 (79·2-v2-3) | 77·1 (69·3·84-9) |
| - At 12 months | 79·4 (70·9-88·0) | 62·9 (51·1-74·8) |
| - At 18 months | 71·2 (59·4-83·0) | 48·6 (28·6 ·68·5) |

| | | |
|---|---|---|
| Abbreviation: CI, confidence interval. | | |

### Additional post-hoc analyses

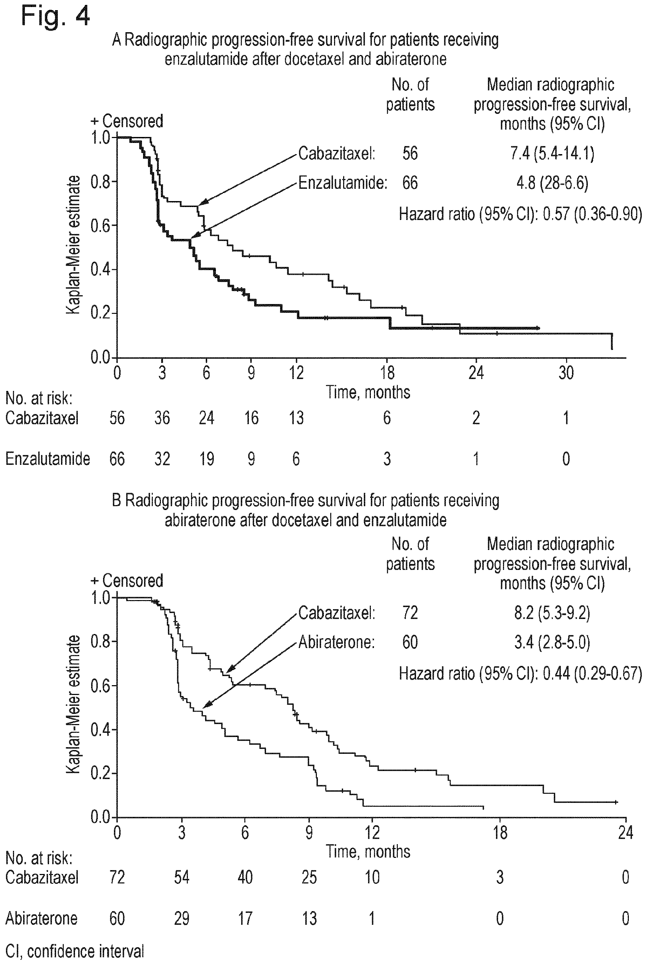

Cabazitaxel remained superior regardless of the androgen signaling targeted inhibitor received (cabazitaxel vs. enzalutamide: hazard ratio, 0.57; 95% Cl, 0.36-0.90; cabazitaxel vs. abiraterone acetate: hazard ratio, 0.44; 95% CI 0.29-0.67) (**Figure 4**). Post hoc multivariate analyses confirmed the robustness of the treatment effect seen in the primary analysis **Table 5**).

**Table 5: Multivariate analysis for radiographic progression-free survival**

| | **Fixed model*** | **Stepwise model selection*** | |
|---|---|---|---|
| | **MV-1^{†}** | **MV-2^{†}** | **MV-3^{¶}** |
| **Prognostic factors as covariates** | | • M1 disease | |
| | | • Hemoglobin, | |
| | | • Lactate dehydrogenase | |
| | | • Alkaline phosphatase | |
| | | • PSA | |
| | • M1 disease | • Duration of first ADT | |
| | • Hemoglobin | • Neutrophil to lymphocyte ratio | |
| | • Lactate dehydrogenase | • Neutrophil count | |
| | • Alkaline phosphatase | • Age | |
| | | • Visceral metastases | |
| | | • Gleason score 8-10 at diagnosis | |
| | | • Prior therapy with curative intent | |
| | | • Type of progression | |
| | | • Testosterone | |
| **Covariates in final model** | • M1 disease | • PSA | • Neutrophil to lymphocyte ratio |
| | • Hemoglobin | | |
| | • Lactate dehydrogenase | | • M1 disease |
| | • Alkaline phosphatase | | • Hemoglobin |

| **Treatment effect** | | | |
|---|---|---|---|
| Hazard ratio | 0.54 | 0.50 | 0.52 |
| (95% CI) | (0.40-0.74) | (0.35-0.71) | (0.36-0.75) |
| P value | < 0.001 | < 0.001 | < 0.001 |

| | | | |
|---|---|---|---|
| * Post-hoc stratified multivariate Cox regression analyses were performed to test the robustness of the primary analysis on radiographic progression-free survival. All the models included three stratification factors. A stepwise model selection approach was used for MV-2 and MV-3, with a significance level of 0.10 for entry into the model and 0.05 for removal a factor from the model. † Prognostic factors were categorical, as specified in the SAP. ¶ If a prognostic factor was collected as continuous, then the continuous variable was used in the model. A log10 transformation was used for PSA, lactate dehydrogenase and alkaline phosphatase. ADT, androgen-deprivation therapy; CI, confidence interval; MV, multivariate; PSA, prostate-specific antigen. | | | |

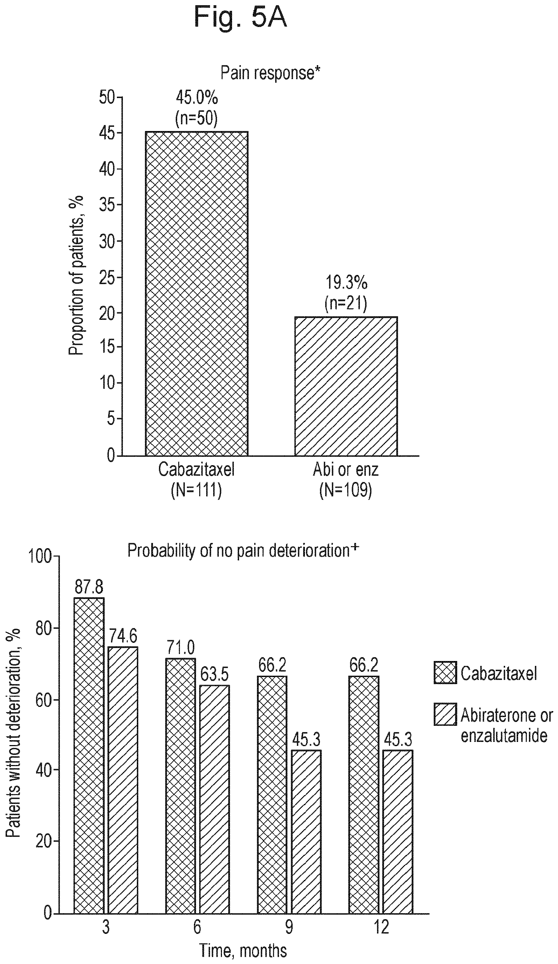

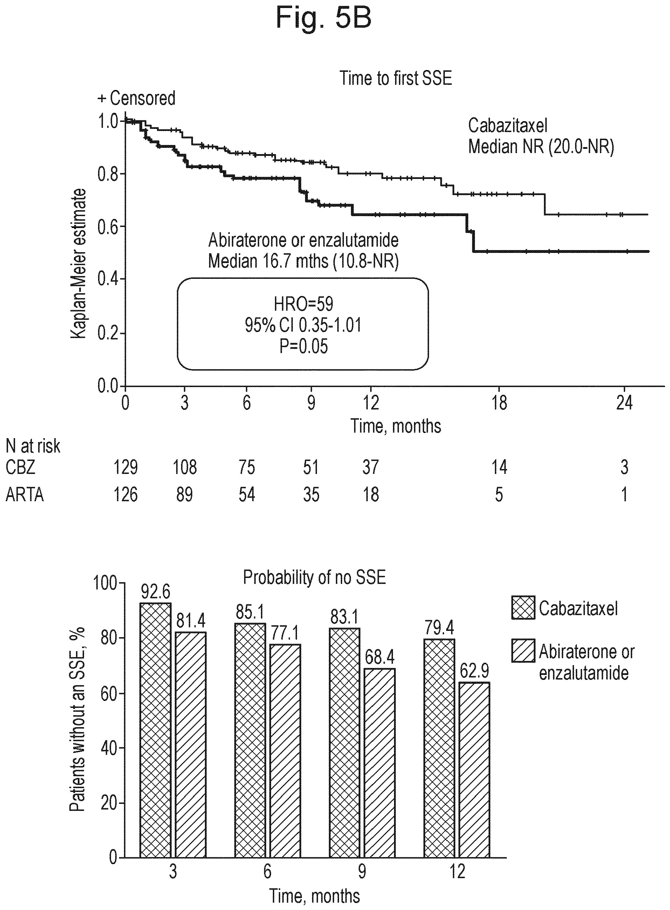

Time to SSE was longer with cabazitaxel compared with an AR-targeted agent (median NR vs 16.7 months; Figure 5B).

### Pain response

Of the 255 patients randomized, 172 (67.5%) had moderate to severe pain at randomization. Pain response and HRQL were evaluable for 111 (86.0%) and 108 (83.7%) for cabazitaxel and 109 (86.5%) and 114 (90.5%) for abiraterone or enzalutamide. Pain response was 45.0% vs 19.3% for cabazitaxel vs abiraterone or enzalutamide (p < 0.0001). The probability of not having pain progression after 12 months was 66.2% vs 45.3% cabazitaxel vs abiraterone or enzalutamide (HR 0.55; 95% Cl 0.32-0.97; p = 0.0348).

An improvement in total FACT-P score from baseline was reported by 27 (25.0%) patients vs 26 (22.8%) for cabazitaxel vs abiraterone or enzalutamide. FACT-P score was maintained or improved for 81 (75.0%) patients with cabazitaxel and 86 (75.4%) patients with abiraterone or enzalutamide. A deterioration in FACT-P from baseline was reported by 22.2% with cabazitaxel vs 24.6% with abiraterone or enzalutamide. Median time to FACT-P deterioration was 14.8 months for cabazitaxel vs 8.9 months for abiraterone or enzalutamide (**Figure 6A**) (HR 0.72; 95% Cl 0.44-1.20; p = 0.2072). Mean changes in FACT-P total score over time were similar for both groups (**Figure 6B**).

Rates of improvement and deterioration were similar between groups (**Figure 6B**).
FACT-P was evaluable in 108/129 patients (83.7%) receiving cabazitaxel and 114/126 patients (90.5%) receiving abiraterone or enzalutamide. Data are shown for up to eight cycles and end of treatment due to the small patient numbers for other cycles. At least one item in the FACT-P questionnaire was completed by ≥ 88·1% of patients in each treatment arm at each visit. At baseline, patients who received cabazitaxel had a mean FACT-P total score of 103·8 versus 105·4 for patients who received abiraterone or enzalutamide (**Table 1**). In repeated-analysis measures using a mixed-effect model, mean changes from baseline in FACT-P total score favored cabazitaxel until Cycle 5 (least squares mean difference ranging from +2·67 to +5·21) and at the end of treatment visit (mean difference of +4·58), but remained comparable with abiraterone or enzalutamide from Cycle 6 to 8.

For FACT-P subscale scores, time to deterioration was numerically longer in patients receiving cabazitaxel compared with patients receiving abiraterone or enzalutamide, although significance was reached only for the emotional well-being subscale. Mean changes from baseline in pain-related subscale scores numerically favored cabazitaxel at all cycles (least squares mean difference ranging from +1·12 to +2·26) and at the end of treatment visit (mean difference of +0·62). The percentage of patients with improved pain-related subscale score was also numerically higher at all visits with cabazitaxel. For Functional Assessment of Cancer Therapy-General (FACT-G), trial outcome index, physical, emotional, and functional well-being, and prostate-specific concerns, mean changes from baseline favored cabazitaxel until Cycle 5, then remained comparable to abiraterone or enzalutamide. Conversely, changes in social or family well-being favored abiraterone or enzalutamide at all cycles (mean difference ranging from -0·37 to -2·25) and at the end of treatment visit (mean difference of -0·94).

**Table 6 Median time to pain progression and deterioration of FACT-P total and subscale scores**

| | Median time to deterioration, months (95% CI) n/nT events, (%) | | | Kaplan-Meier probability of no deterioration at 3 months (95% CI) | |
|---|---|---|---|---|---|
| | Cabazitaxel | Abiraterone or enzalutamide | HR (95% CI) | Cabazitaxel | Abiraterone or Enzalutamide |
| Pain (BPI-SF) | NE (NE-NE) 25/111 (22·5) | 8·5 (4·9-NE) 27/109 (24·8) | 0·55* (0·32-0·97) | 87·8 (81·3-94·3) | 74·6 (64·9-84·3) |
| FACT-P total | 14·8 (6·3-NE) 32/108 (29·6) | 8·9 (6·3-NE) 33/114 (28·9) | 0·72 (0·44-1·20) | 86·2 (79·5-93·0) | 74·0 (65·2-82·7) |
| FACT-G total | 14·8 (8·8-NE) 30/108 (27·8) | 11·4 (11·4-NE) 32/114 (28·1) | 0·71 (0·42-1·18) | 82·3 (74·9-89·8) | 72·3 (63·2-81·4) |
| Trial outcome index | 14·8 (8·5-NE) 29/108 (26·9) | 8·9 (6·3-NE) 34/114 (29·8) | 0·65 (0·39-1·09) | 86·1 (79·3-92·9) | 71·4 (66·2-80·6) |
| Physical well-being | 14·8 (4·9-NE) 39/108 (36·1) | 8·9 (4·3-NE) 38/114 (33·3) | 0·82 (0·51-1·30) | 76·0 (67·5-84·4) | 71·4 (62·4-80·4) |
| Social or family well-being | 14·8 (7·9-14·8) 35/108 (32v·4) | 8·9 (6·3-NE) 27/114 (23·7) | 1·03 (0·61-1·73) | 78·7 (70·8-86·7) | 79·4 (71·2-87·6) |
| Emotional well-being | NE (NE-NE) 18/108 (16·7) | 13·7 (6·3-NE) 26/114 (22·8) | 0·46** (0·25-0·87) | 91.1 (85·5-96·7) | 8·26 (74·9-90·3) |
| Functional well-being | NE (5·9-NE) 39/108 (36·1) | 8·9 (4·8-NE) 39/114 (34·2) | 0·81 (0·51-1·28) | 73·7 (65·2-82·3) | 65·3 (55·7-74·9) |
| Prostate-specific concerns | 14·8 (9-8-NE) 35/108 (32-4) | 8·9 (4-8-NE) 40/114 (35-1) | 0·68 (0·42-1·08) | 83·6 (76·4-90·7) | 68·4 (59·1-77·6) |
| Pain-related subscale | 10·4 (8·5-NE) 36/108 (33·3) | 8·9 (4·9-NE) 38/114 (33·3) | 0·74 (0·46-1·19) | 81·6 (74·1-89·1) | 69·6 (60·5-78·7) |

| | | | | | |
|---|---|---|---|---|---|
| Stratified log rank test: *p=0·03; **p=0·015 Abbreviations: BPI-SF, Brief Pain Inventory-Short Form, CI, confidence interval; FACT-G, Functional Assessment of Cancer Therapy-General; FACT-P, Functional Assessment of Cancer Therapy-Prostate; HR, hazard ratio; NE, not estimable. | | | | | |

### Patient-reported outcomes (PROs)

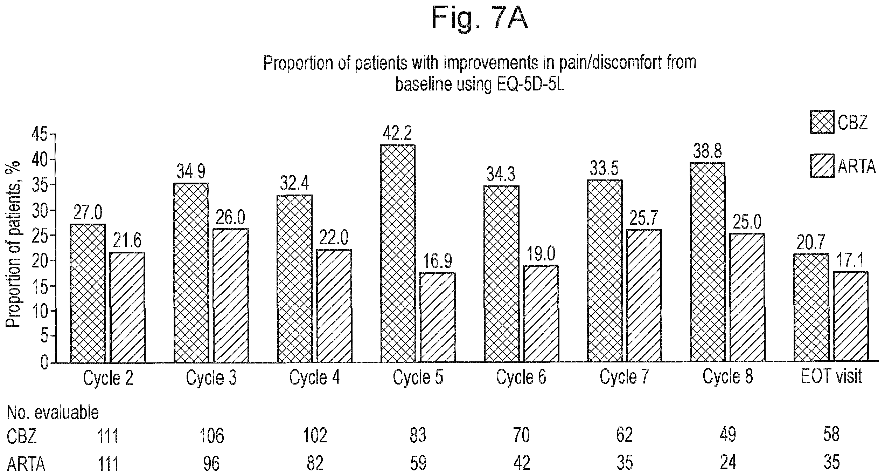

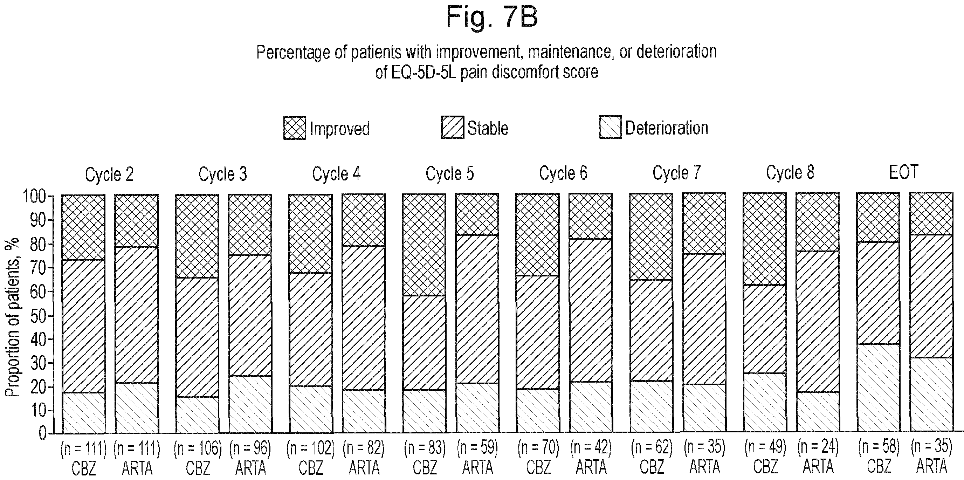

Of the 255 patients randomized, 230 were evaluable for EQ-5D-5L, with 115 (89.1%) for cabazitaxel and 115 (91.3%) for abiraterone or enzalutamide. At baseline, both groups had comparable mean values for utility index (0.70 for both) and VAS (65.8 vs 66.3 for CBZ vs abiraterone or enzalutamide, respectively-**table 1**). Changes in VAS and utility index scores from baseline during treatment and EOT favored cabazitaxel vs abiraterone or enzalutamide. The least squares mean difference for cabazitaxel vs abiraterone or enzalutamide ranged 1.6 to 6.4 (5.9 at EOT) for VAS and from 0.03 to 0.08 and (0.05 at EOT) for utility index. At baseline, moderate to severe or extreme pain/discomfort, evaluated by EQ-5D-5L, was reported by 45 (39.1%) for cabazitaxel and 47 (40.9%) for abiraterone or enzalutamide, respectively. cabazitaxel was associated with a greater proportion of patients reporting pain/discomfort improvement from baseline (**figure 7**). Similar trends were seen across all EQ-5D-5L subscales.

EQ-5D-5L was evaluable in 115/129 patients (89.1%) receiving cabazitaxel and 115/126 patients (91.3%) receiving abiraterone or enzalutamide. Data are shown for up to 8 cycles and end of treatment because the number of patients receiving subsequent cycles in the control arm was too small. At least one item in the EQ-5D-5L questionnaire was completed by ≥ 82.4% of patients in each treatment arm at each visit. Mean values for EQ-5D-5L health status (visual analogue score; VAS) at baseline were 65.8 for cabazitaxel and 66.3 for abiraterone or enzalutamide. EQ-5D-5L mean change from baseline in VAS favored cabazitaxel over abiraterone or enzalutamide, with least squares mean difference ranging from +1·6 to +6·4 during treatment and +5·9 at end of treatment (**Figure 6C**).

Mean change from baseline in utility score favored cabazitaxel over abiraterone or enzalutamide, with least squares mean difference ranging from +0·03 to +0·08 during treatment and +0·05 at end of treatment (**Figure 6C**).

Moderate, severe, or extreme pain/discomfort was reported by 45 patients (39·1%) receiving cabazitaxel and 47 patients (40·9%) receiving abiraterone or enzalutamide at baseline. Cabazitaxel was associated with a greater proportion of patients reporting improvement in pain/discomfort from baseline. Similar trends were seen for other EQ-5D-5L subscales during treatment with cabazitaxel.

### Safety

Almost all patients experienced an adverse event of any grade in both treatment arms (98.4% vs. 94.4% for cabazitaxel vs. the androgen signaling targeted inhibitor, respectively) (**Table 7**). Serious adverse event rates of any grade were similar for cabazitaxel (38.9%) and the androgen signaling targeted inhibitor (38.7%). Adverse events leading to treatment discontinuation occurred more frequently with cabazitaxel (19.8%) vs. an androgen signaling targeted inhibitor (8.9%). Conversely, adverse events leading to death during the treatment emergent adverse event period (from randomization to 30 days after last treatment administration) occurred more frequently with the androgen signaling targeted inhibitor (14 patients, 11.3%) vs. cabazitaxel (seven patients, 5.6%). The grade 5 adverse events reported in the cabazitaxel arm were related to infections (two patients), bronchial aspiration (one patient), general health deterioration due to progressive disease (two patients), spinal cord compression (one patient) and head injury (one patient). Grade 5 adverse events in the androgen signaling targeted inhibitor arm were related to infections (two patients), pulmonary thromboembolism (one patient), cardiac disorders (two patients), cerebral bleeding associated with hyperfibrinolysis (one patient), renal failure (two patients) and general health deterioration due to progressive disease (six patients) associated in one case with upper gastrointestinal bleeding, hypertensive crisis and cardiac failure.

Grade ≥3 adverse events occurring more frequently with cabazitaxel than the androgen signaling targeted inhibitors were asthenia/fatigue (4.0% vs 2.4%), diarrhea (3.2% vs. 0%), peripheral neuropathy (3.2% vs. 0%) and febrile neutropenia (3.2% vs. 0%). Grade ≥3 occurring more frequently with the androgen signaling targeted inhibitors than cabazitaxel were renal disorders (8.1% vs 3.2%), musculoskeletal pain/discomfort (5.6% vs 1.6%), cardiac disorders (4.8% vs. 0.8%) and spinal cord/nerve root disorders (4.0% vs 2.4%) (**Table 7**). Grade ≥3 neutropenia, measured at nadir by blood test, was observed in 55 patients (43.7%) with cabazitaxel. In the cabazitaxel arm, any grade hematuria was reported by 19 patients (15.1%), mild alopecia by seven patients (5.6%) and no nail disorders were reported. No new safety signals were reported.

At least one dose reduction was required in 27 patients (21.4%) receiving cabazitaxel and 47 patients (37.9%) receiving an androgen signaling targeted inhibitor (abiraterone acetate: 17 [29.3%]; enzalutamide: 30 [45.5%])

**Table 7 Adverse events**

| **Patients, n (%)** | **Cabazitaxel N = 126** | | **Androgen signaling targeted inhibitor N = 124** | |
|---|---|---|---|---|
| Any adverse events | 124 (98.4) | | 117 (94.4) | |
| Any grade ≥3 adverse events | 71 (56.3) | | 65 (52.4) | |
| Any serious adverse events | 49 (38.9) | | 48 (38.7) | |
| Any adverse event leading to permanent treatment discontinuation | 25 (19.8) | | 11 (8.9) | |
| Any adverse event leading to death* | 7 (5.6) | | 14 (11.3) | |

| | **All grades** | **Grade ≥ 3** | **All grades** | **Grade ≥ 3** |
|---|---|---|---|---|
| **Adverse events of any grade reported in ≥5% and/or grade ≥3 reported in ≥3% of patients for either treatment arm, n (%)** | | | | |
| Asthenia/fatigue | 67 (53.2) | 5 (4.0) | 45 (36.3) | 3 (2.4) |
| Diarrhea | 50 (39.7) | 4 (3.2) | 8 (6.5) | 0 |
| Infections and infestations | 40 (31.7) | 10 (7.9) | 25 (20.2) | 9 (7.3) |
| Musculoskeletal pain/discomfort^{†} | 34 (27.0) | 2 (1.6) | 49 (39.5) | 7 (5.6) |
| Nausea/vomiting | 33 (26.2) | 0 | 29 (23.4) | 2 (1.6) |
| Peripheral neuropathy | 25 (19.8) | 4 (3.2) | 4 (3.2) | 0 |
| Constipation | 19 (15.1) | 0 | 13 (10.5) | 0 |
| Hematuria | 19 (15.1) | 1 (0.8) | 7 (5.6) | 2 (1.6) |
| Decreased appetite | 17 (13.5) | 1 (0.8) | 19 (15.3) | 3 (2.4) |
| Dysgeusia | 14 (11.1) | 0 | 5 (4.0) | 0 |
| Bladder and urethral symptoms^{‡} | 12 (9.5) | 0 | 10 (8.1) | 0 |
| Abdominal pain | 10 (7.9) | 1 (0.8) | 3 (2.4) | 1 (0.8) |
| Stomatitis | 10 (7.9) | 0 | 2 (1.6) | 0 |
| Peripheral edema | 10 (7.9) | 0 | 11 (8.9) | 1 (0.8) |
| Renal disorders^{§} | 8 (6.3) | 4 (3.2) | 14 (11.3) | 10 (8.1) |
| Cardiac disorders | 8 (6.3) | 1 (0.8) | 10 (8.1) | 6 (4.8) |
| Arthralgia | 8 (6.3) | 0 | 16 (12.9) | 1 (0.8) |
| Dyspnea | 7 (5.6) | 0 | 3 (2.4) | 0 |
| Alopecia | 7 (5.6) | 0 | 0 | 0 |
| Spinal cord and nerve root disorders" | 6 (4.8) | 3 (2.4) | 9 (7.3) | 5 (4.0) |
| Psychiatric disorders^{¶} | 5 (4.0) | 0 | 15 (12.1) | 0 |
| Hypertensive disorders | 5 (4.0) | 3 (2.4) | 10 (8.1) | 3 (2.4) |
| Weight decreased | 5 (4.0) | 0 | 7 (5.6) | 0 |
| Febrile neutropenia | 4 (3.2) | 4 (3.2) | 0 | 0 |
| Bone fractures | 3 (2.4) | 1 (0.8) | 7 (5.6) | 2 (1.6) |

| **Laboratory abnormalities, n/N (%)^{◊}** | | | | |
|---|---|---|---|---|
| Anemia | 124/125 (99.2) | 10/125 (8.0) | 118/124 (95.2) | 6/124 (4.8) |
| Leukopenia | 93/125 (74.4) | 40/125 (32.0) | 39/124 (31.5) | 2/124 (1.6) |
| Neutropenia | 81/123 (65.9) | 55/123 (44.7) | 8/124 (6.5) | 4/124 (3.2) |
| Thrombocytopenia | 51/125 (40.8) | 4/125 (3.2) | 20/124 (16.1) | 2/124 (1.6) |
| Increased aspartate transferase | 27/124 (21.8) | 4/124 (3.2) | 35/124 (28.2) | 0 |
| Increased alanine transferase | 24/124 (19.4) | 1/124 (0.8) | 11/124 (8.9) | 0 |
| Hypokalemia | 15/125 (12.0) | 1/125 (0.8) | 19/124 (15.3) | 1/124 (0.8) |
| | | | | |

| | | | | |
|---|---|---|---|---|
| *During the treatment emergent adverse event period (from randomization to 30 days after last treatment administration); † Includes back pain, flank pain, musculoskeletal discomfort and pain, neck pain, pain in extremities; ‡ Includes dysuria, pollakiuria, lower urinary tract symptoms, micturition urgency, urinary incontinence and urinary retention; § Includes, acute kidney injury, renal failure and impairment, hydronephrosis, pyelocaliectasis; ∥ Includes sciatalgia, radiculopathy, spinal cord compression; ¶ Include anxiety, depression, confusion, disorientation, sleep disorders; ◊ Laboratory abnormalities are based on systematic analysis of blood samples at each cycle (may not have been reported as an adverse event). | | | | |

### First anticancer treatment after progression

Of 126 patients receiving an androgen signaling targeted inhibitor, 42 (33.3%) crossed over to cabazitaxel. Of 129 patients receiving cabazitaxel, 30 patients (23.3%) crossed over to abiraterone acetate or enzalutamide (**Table 8**).

**Table 8: first subsequent anti-cancer treatment**

| | **Cabazitaxel N = 129** | **AR pathway inhibitor N** = **126** |
|---|---|---|
| **Patients, n (%)** | | |
| Docetaxel | 2 (1.6) | 1 (0.8) |
| Abiraterone | 15 (11.6) | 0 (0) |
| Enzalutamide | 15 (11.6) | 2 (1.6) |
| Cabazitaxel | 3 (2.3) | 42 (33.3) |
| Radium 223 | 6 (4.7) | 3 (2.4) |
| Investigational drug | 6 (4.7) | 3 (2.4) |
| Taxane combined with platinum agent | 3 (2.3) | 2 (1.6) |
| Palliative radiation therapy | 19 (14.7) | 28 (22.2) |

The results of this study demonstrate that fit patients with mCRPC who have previously been treated with docetaxel and progressed within 12 months on an alternative androgen signaling targeted inhibitor experience longer imaging-based progression-free survival and overall survival when treated with cabazitaxel compared with abiraterone acetate or enzalutamide.

Cabazitaxel more than doubled imaging-based progression-free survival (median 8.0 vs 3.7 months) and this benefit was observed across all pre-specified subgroups, irrespective of the previous alternative androgen signaling targeted inhibitor timing, before or after docetaxel. Cabazitaxel reduced the risk of death from any cause by 36% compared with abiraterone acetate or enzalutamide, despite 33% of patients crossing over to cabazitaxel at the time of progression on the androgen signaling targeted inhibitor. All other secondary end points (progression-free survival, PSA response, tumor response, pain response and time to symptomatic skeletal events) also favored cabazitaxel.

The incidence of grade ≥3 adverse events was comparable in both treatment arms (56.3% with cabazitaxel, 52.4% with the androgen signaling targeted inhibitors). Adverse events leading to death during the study were doubled with an androgen signaling targeted inhibitor versus cabazitaxel, mainly related to disease progression. Neutropenic complications with cabazitaxel usually occur at Cycle 1, especially when the baseline neutrophil count is below 4000/mm. Therefore, all patients received prophylactic granulocyte colony-stimulating factor during every cycle in the study.

No preplanned analysis of the influence of abiraterone-enzalutamide (or vice versa) sequence was undertaken. However, since retrospective studies suggest the sequence of androgen signaling targeted inhibitors may influence progression-free survival, post hoc analyses were performed confirming the superiority of cabazitaxel over the androgen signaling targeted inhibitor regardless of whether abiraterone or enzalutamide was received

In conclusion, cabazitaxel led to an improvement in imaging-based progression-free survival compared with abiraterone acetate or enzalutamide in patients with mCRPC who had received docetaxel and an alternative androgen signaling targeted inhibitor. Cabazitaxel also significantly improved overall survival and other secondary end points.

Cabazitaxel was also associated with significantly improved pain response (45.0% versus 19.3%; p<0.0001) and prolonged time without pain progression versus abiraterone or enzalutamide. Cabazitaxel and abiraterone or enzalutamide were associated with similar trends in HRQL but time to FACT-P deterioration was longer with cabazitaxel when compared to abiraterone or enzalutamide, especially emotional well-being, pain-related subscale, trial outcome index, and prostate-specific concerns, although social or family well-being favored the androgen-signaling-targeted inhibitors. Cabazitaxel showed also a greater improvement in PROs measured by EQ-5D-5L vs abiraterone or enzalutamide in patients previously treated with docetaxel and the alternative AR-targeted agent.

Cabazitaxel also reduced the probability of developing SSEs (71.2% versus 48.6% at 18 months) despite a lower use of denosumab or bisphosphonates compared with patients receiving abiraterone or enzalutamide.

## Claims

1. Cabazitaxel, or a hydrate or solvate thereof, for use in a method of increasing progression free survival in a patient in need thereof, comprising administering an effective amount of cabazitaxel, or a hydrate or solvate thereof, to said patient having metastatic castration-resistant prostate cancer previously treated with docetaxel and who failed a prior AR-targeted agent.

2. Cabazitaxel, or a hydrate or solvate thereof, for use in a method of increasing overall survival in a patient in need thereof, comprising administering an effective amount of cabazitaxel, or a hydrate or solvate thereof, to said patient having metastatic castration-resistant prostate cancer previously treated with docetaxel and who failed a prior AR-targeted agent.

3. Cabazitaxel, or a hydrate or solvate thereof, for use in the reduction or removal of pain in a patient having metastatic castration-resistant prostate cancer previously treated with docetaxel and who failed a prior AR-targeted agent.

4. Cabazitaxel, or a hydrate or solvate thereof, in combination with a corticoid for use as a medicament in the treatment of patients having metastatic castration-resistant prostate cancer previously treated with docetaxel and who failed a prior AR-targeted agent.

5. Cabazitaxel or a hydrate or solvate thereof according to any one of claims 1 to 3, wherein cabazitaxel, or hydrate or solvate thereof, is administered in combination with a corticoid.

6. Cabazitaxel or a hydrate or solvate thereof according to any one of claims 1 to 5, wherein cabazitaxel, or hydrate or solvate thereof, is administered prior to the administration of an alternative AR-targeted agent.

7. Cabazitaxel or a hydrate or solvate thereof according to any one of claims 1 to 6, wherein an alternative AR-targeted agent is administered following disease progression after cabazitaxel treatment.

8. Cabazitaxel or a hydrate or solvate thereof according to anyone of claims 1 to 7, wherein said patient has not previously received an alternative AR-targeted agent.

9. Cabazitaxel or a hydrate or solvate thereof according to any one of claims 1 to 8, wherein the prior AR-targeted agent is abiraterone acetate and the alternative AR-targeted agent is enzalutamide.

10. Cabazitaxel or a hydrate or solvate thereof according to any one of claims 1 to 9, wherein the prior AR-targeted agent is enzalutamide and the alternative AR-targeted agent is abiraterone acetate.

11. Cabazitaxel or a hydrate or solvate thereof according to any one of claims 1 to 10, wherein the failure of a prior AR-targeted agent occurred within 12 months.

12. Cabazitaxel or a hydrate or solvate thereof according to any one of claims 1 to 11, wherein the corticoid is prednisone or prednisolone.

13. Cabazitaxel or a hydrate or solvate thereof according to claim 12, wherein the prednisone or prednisolone is administered at a dose of 10 mg/day.

14. Cabazitaxel or a hydrate or solvate thereof according to any one of claims 1 to 13, wherein an effective amount of cabazitaxel, or hydrate or solvate thereof is 15 to 25 mg/m².

15. Cabazitaxel, or a hydrate or solvate thereof according to any one of claims 1 to 14, wherein the cabazitaxel is in a form of an acetone solvate.

16. Cabazitaxel, or a hydrate or solvate thereof according to any one of claims 1 to 15, wherein the cabazitaxel, or hydrate or solvate thereof, is administered at a dose of 2 25 mg/m .

17. Cabazitaxel, or a hydrate or solvate thereof according to any one of claims 1 to 15, wherein the cabazitaxel, or hydrate or solvate thereof, is administered at a dose of 20 mg/m².

18. Cabazitaxel, or a hydrate or solvate thereof according to any one of claims 1 to 15, wherein the cabazitaxel, or hydrate or solvate thereof, is administered at a dose of 15 mg/m².

19. Cabazitaxel, or a hydrate or solvate thereof according to any one of claims 1 to 18, wherein the method comprises repeating the administration of cabazitaxel, or hydrate or solvate thereof, as a new cycle every 3 weeks.

20. Cabazitaxel, or a hydrate or solvate thereof according to any one of claims 1 to 19, wherein the patient having metastatic castration-resistant prostate cancer is a patient aged 70 years or over.

21. Cabazitaxel, or a hydrate or solvate thereof according to any one of claims 1 to 20, wherein the patient has PSA progression only or imaging-based progression with or without PSA progression, at the time of initiation of cabazitaxel administration.

22. Cabazitaxel, or a hydrate or solvate thereof according to any one of claims 1 to 21, wherein the cabazitaxel, or hydrate or solvate thereof is administered in combination with G-CSF.
